# EUROPÄISCHE PATENTSCHRIFT

(11) **EP 1 254 133 B1**
(45) Veröffentlichungstag und Bekanntmachung des Hinweises auf die Patenterteilung: **20.04.2005**
(21) Anmeldenummer: 01903624.3
(22) Anmeldetag: 05.01.2001
(51) Int. Cl.: C07D 401/12, A61K 31/404, A61P 43/00, C07D 403/12, C07D 417/14, C07D 209/18

(54) **INDOL-3-YL-DERIVATE UND DEREN VERWENDUNG ALS INTEGRIN-INHIBITOREN**
INDOL-3-YL DERIVATIVES AND THEIR USE AS INTEGRIN INHIBITORS
DERIVES DE 3-INDOLE ET LEUR UTILISATION COMME INHIBITEURS D'INTEGRINE

(30) Priorität: 11.02.2000 DE 10006139
(43) Veröffentlichungstag der Anmeldung: 06.11.2002
(73) Patentinhaber: MERCK PATENT GmbH, 64293 Darmstadt (DE)
(72) Erfinder: GOODMAN, Simon, 64347 Griesheim (DE); GOTTSCHLICH, Rudolf, 64354 Reinheim (DE); WIESNER, Matthias, 55128 Mainz (DE)
(86) Internationale Anmeldenummer: PCT/EP2001/000084
(87) Internationale Veröffentlichungsnummer: WO 2001/058893

(56) Entgegenhaltungen:
- WO-A-94/12478
- WO-A-97/37655
- WO-A-99/33798

## Beschreibung

Die Erfindung betrifft Indol-3-yl-Derivate der Formel 1 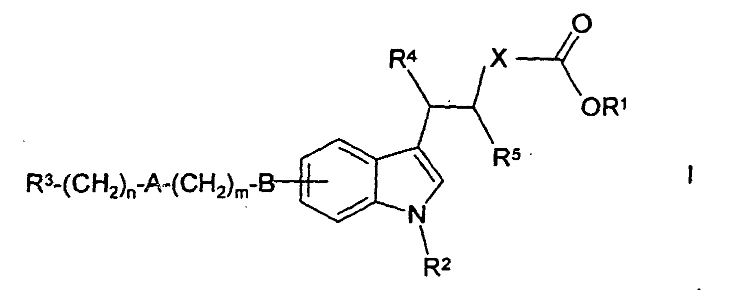 worin
- A: NH, CONH, NHCO oder eine direkte Bindung sein kann,
- B: O ist,
- X: eine direkte Bindung,
- R¹: H,
- R²: H,
- R³: 1H-Imidazol-2-yl, 4,5-Dihydro-imidazol-2-yl, 3,5-Dihydro-imidazol-4-on-2-yl oder Pyridin-2-yl, wobei diese jeweils ein- oder zweifach durch =O oder NHZ substituiert sein können,
- R⁴: Phenyl, 3-Trifluormethoxyphenyl, 4-Fluorphenyl, 3-Chlorphenyl, 3-Hydroxyphenyl, Pyridin-4-yl, 3,5-Dichlorphenyl, 2,4-Dichlorphenyl, Cyclohexyl, 4-Chlor-3-trifluormethylphenyl, Benzothiadiazol-5-yl, 2,6-Difluorphenyl, 2-Chlor-3,6-difluorphenyl, 2,4,6-Trifluorphenyl oder Cyclohexyl
- R⁵: H,
- Z: Alkyl mit 1 bis 6 C-Atomen,
- n: 0,
- m: 3 oder 4
- o: 0, 1 oder 2
bedeuten,
sowie deren physiologisch unbedenklichen Salze und Solvate.

Teilweise ähnliche Verbindungen sind aus WO 99/30713 oder WO 94/12478 bekannt.

Der Erfindung lag die Aufgabe zugrunde, neue Verbindungen mit wertvollen Eigenschaften aufzufinden, insbesondere solche, die zur Herstellung von Arzneimitteln verwendet werden.

Es wurde gefunden, daß die Verbindungen der Formel I und ihre Salze bei guter Verträglichkeit sehr wertvolle pharmakologische Eigenschaften besitzen. Vor allem wirken sie als Integrin-Inhibitoren, wobei sie insbesondere die Wechselwirkungen der αv-, β3- oder β5-Integrin-Rezeptoren mit Liganden hemmen, wie z.B. die Bindung von Vitronektin an den Integrinrezeptor. Integrine sind membrangebundene, heterodimere Glycoproteine, die aus einer α-Untereinheit und einer kleineren β-Untereinheit bestehen. Die relative Affinität und Spezifität für eine Ligandenbindung wird durch Kombination der verschiedenen α- und β-Untereinheiten bestimmt. Besondere Wirksamkeit zeigen die erfindungsgemäßen Verbindungen im Fall der Integrine αvβ1, αvβ3, αvβ5, αIIbβ3 sowie αvβ6 und αvβ8, bevorzugt von αvβ3 und αvβ5, sowie αIIbβ3. Die erfindungsgemäßen Verbindungen sind insbesondere potente Inhibitoren des Vitronektinrezeptors αvβ3 und/oder αvβ5 und/oder des Fibrinogenrezeptors αIIbβ3. Die erfindungsgemäßen Verbindungen sind besonders bevorzugt Inhibitoren des Vitronektinrezeptors αvβ3.

Essentiell für die Aktivität von Integrininhibitoren ist die Anwesenheit einer Säurefunktion in einem geeigneten Abstand zu einem Basenzentrum.
Durch Anpassung der Spacerlänge und Art des Basenzentrums kann die Aktivität und Spezifität gesteuert werden. Als zentrales Templat eignet sich Indol.

Das αvβ3 Integrin wird auf einer Reihe von Zellen, z.B. Endothelzellen, Zellen der glatten Gefäßmuskulatur beispielsweise der Aorta, Zellen zum Abbau von Knochenmatrix (Osteoclasten) oder Tumorzellen, exprimiert.

Die Wirkung der erfindungsgemäßen Verbindungen kann z.B. nach der Methode nachgewiesen werden, die von J.W. Smith et al. in J. Biol. Chem. **1990**, *265*, 12267-12271 beschrieben wird.
B. Felding-Habermann und D.A. Cheresh beschreiben in Curr. Opin. Cell. Biol. **1993**, *5*, 864 die Bedeutungen der Integrine als Adhäsionsrezeptoren für die unterschiedlichsten Phänomene und Krankheitsbilder, speziell in Bezug auf den Vitronectinrezeptor αvβ3.
Die Abhängigkeit der Entstehung von Angiogenese von der Wechselwirkung zwischen vaskulären integrinen und extrazellulären Matrixproteinen ist von P.C. Brooks, R.A. Clark und D.A. Cheresh in Science **1994,** *264*, 569-571 beschrieben.

Die Möglichkeit der Inhibierung dieser Wechselwirkung und damit zum Einleiten von Apoptose (programmierter Zelltod) angiogener vaskufärer Zellen durch ein cyclisches Peptid ist von P.C. Brooks, A.M. Montgomery, M. Rosenfeld, R.A. Reisfeld, T. Hu, G. Klier und D.A. Cheresh in Cell **1994,** *79*, 1157-1164 beschrieben. Es wurden darin z.B. αvβ3-Antagonisten oder Antikörper gegen αvβ3 beschrieben, die eine Schrumpfung von Tumoren durch Einleiten von Apoptose bewirken.

Der experimentelle Nachweis, daß auch die erfindungsgemäßen Verbindungen die Anheftung von lebenden Zellen auf den entsprechenden Matrixproteinen verhindern und dementsprechend auch die Anheftung von Tumorzellen an Matrixproteine verhindern, kann in einem Zelladhäsionstest erbracht werden, analog der Methode von F. Mitjans et al., J. Cell Science **1995**, *108*, 2825-2838.

P.C. Brooks et al. beschreiben in J. Clin. Invest. **1995**, *96*, 1815-1822 αᵥβ₃-Antagonisten zur Krebsbekämpfung und zur Behandlung tumor-induzierter angiogener Krankheiten.
Die Verbindungen können die Bindung von Metallproteinasen an Integrine hemmen und so verhindern, daß die Zellen die enzymatische Aktivität der Proteinase nutzen können. Ein Beispiel ist in der Hemmbarkeit der Bindung von MMP-2- (Matrix-Metallo-Proteinase-2-) an den Vitronektin-Rezeptor αvβ3 durch ein Cyclo-RGD-Peptid zu finden, wie in P.C. Brooks et al., Cell **1996**, *85*, 683-693 beschrieben.
Die erfindungsgemäßen Verbindungen der Formel I können daher als Arzneimittelwirkstoffe insbesondere zur Behandlung von Tumorerkrankungen, Osteoporosen, osteolytischen Erkrankungen sowie zur Unterdrückung der Angiogenese eingesetzt werden.

Verbindungen der Formel I, die die Wechselwirkung von Integrinrezeptoren und Liganden, wie z.B. von Fibrinogen an den Fibrinogenrezeptor (Glycoprotein IIb/IIIa oder αIIβ3) blockieren, verhindern die Ausbreitung von Tumorzellen durch Metastase und können daher als antimetastatisch wirkende Substanzen bei Operationen eingesetzt werden, bei denen Tumore chirurgisch entfernt oder angegriffen werden. Dies wird durch folgende Beobachtungen belegt:

Die Verbreitung von Tumorzellen von einem lokalen Tumor in das vaskuläre System erfolgt durch die Bildung von Mikroaggregaten (Mikrothromben) durch die Wechselwirkung der Tumorzellen mit Blutplättchen. Die Tumorzellen sind durch den Schutz im Mikroaggregat abgeschirmt und werden von den Zellen des Immunsystems nicht erkannt. Die Mikroaggregate können sich an Gefäßwandungen festsetzen, wodurch ein weiteres Eindringen von Tumorzellen in das Gewebe erleichtert wird. Da die Bildung der Mikrothromben durch Ligandenbindung an die entsprechenden Integrinrezeptoren, z.B. αvβ3 oder αIIbβ3, auf aktivierten Blutplättchen vermittelt wird, können die entsprechenden Antagonisten als wirksame Metastase-Hemmer angesehen werden.

Verbindungen der Formel I hemmen neben der Bindung von Fibrinogen, Fibronectin und des von-Willebrand-Faktors an den Fibrinogenrezeptor der Blutplättchen auch die Bindung weiterer adhäsiver Proteine, wie Vitronectin, Kollagen und Laminin, an die entsprechenden Rezeptoren auf der Oberfläche verschiedener Zelltypen. Sie verhindern insbesondere die Entstehung von Blutplättchenthromben und können daher zur Behandlung von Thrombosen, Apoplexie, Herzinfarkt, Entzündungen und Arteriosklerose eingesetzt werden.

Die thrombozytenaggregationshemmende Wirkung läßt sich in vitro nach der Methode von Born (Nature **1962**, *4832*, 927-929) nachweisen.

Die Verbindungen der Formel I können als Arzneimittelwirkstoffe in der Human- und Veterinärmedizin eingesetzt werden, insbesondere zur Prophylaxe und/oder Therapie von Erkrankungen des Kreislaufs, Thrombose, Herzinfarkt, Arteriosklerose, Apoplexie, Angina pectoris, Tumorerkrankungen, wie Tumorentwicklung oder Tumormetastasierung, osteolytischen Krankheiten wie Osteoporose, pathologisch angiogenen Krankheiten wie z.B. Entzündungen, ophthalmologischen Krankheiten, diabetischer Retinopathie, makularer Degeneration, Myopia, okularer Histoplasmose, Restenose, rheumatischer Arthritis, Osteoarthritis, rubeotischem Glaukom, ulcerativer Colitis, Morbus Crohn, Atherosklerose, Psoriasis, Restenose nach Angioplastie, Multiptesklerose, viraler Infektion, bakterieller Infektion, Pilzinfektion, bei akutem Nierenversagen und bei der Wundheilung zur Unterstützung des Heilungsprozesses.

Die Verbindungen der Formel I können als antimikrobiell wirkende Substanzen bei Operationen eingesetzt werden, wo Biomaterialien, Implantate, Katheter oder Herzschrittmacher verwendet werden. Dabei wirken sie antiseptisch. Die Wirksamkeit der antimikrobiellen Aktivität kann durch das von P. Valentin-Weigund et al. in Infection and Immunity, **1988**, 2851-2855 beschriebene Verfahren nachgewiesen werden.

Ein Maß für die Aufnahme eines Arzneimittelwirkstoffs in einen Organismus ist seine Bioverfügbarkeit.
Wird der Arzneimittelwirkstoff in Form einer Injektionslösung dem Organismus intravenös zugefügt, so liegt seine absolute Bioverfügbarkeit, d.h. der Anteil des Pharmakons, der unverändert im systemischen Blut, d.h. in den großen Kreislauf gelangt, bei 100%.
Bei oraler Vergabe eines therapeutischen Wirkstoffs liegt der Wirkstoff in der Regel als Feststoff in der Formulierung vor und muß sich daher zuerst auflösen, damit er die Eintrittsbarrieren, beispielsweise den Gastrointestinaltrakt, die Mundschleimhaut, nasale Membranen oder die Haut, insbesondere das Stratum corneum, überwinden kann bzw. vom Körper resorbiert werden kann. Daten zur Pharmakokinetik, d.h. zur Bioverfügbarkeit können analog zu der Methode von J. Shaffer et al, J. Pharm. Sciences, 1999, 88, 313-318 erhalten werden.

Gegenstand der Erfindung sind Verbindungen der Formel I nach Anspruch 1 und ihre physiologisch unbedenklichen Salze und/oder Solvate als therapeutische Wirkstoffe.

Gegenstand der Erfindung sind demgemäß Verbindungen der Formel I nach Anspruch 1 und ihre physiologisch unbedenklichen Salze und/oder Solvate als αv-Integrininhibitoren.

Weiterhin sind Gegenstand der Erfindung Verbindungen der Formel I nach Anspruch 1 und ihre physiologisch unbedenklichen Salze und/oder Solvate als GPllb/llla-Antagonisten.

Gegenstand der Erfindung sind Verbindungen der Formel I nach Anspruch 1 und ihre physiologisch unbedenklichen Salze und/oder Solvate zur Anwendung bei der Bekämpfung von Krankheiten.

Die Verbindungen der Formel 1 besitzen mindestens ein chirales Zentrum und können daher in mehreren stereoisomeren Formen auftreten. Alle diese Formen (z.B. D- und L-Formen) und deren Gemische (z.B. die DL-Formen) sind in der Formel eingeschlossen.

In die erfindungsgemäßen Verbindungen nach Anspruch 1 sind auch sogenannte Prodrug-Derivate eingeschlossen, d.h. mit z.B. Alkyl- oder Acylgruppen, Zuckern oder Oligopeptiden abgewandelte Verbindungen der Formel 1, die im Organismus rasch zu den wirksamen erfindungsgemäßen Verbindungen gespalten werden.
Ferner können freie Aminogruppen oder freie Hydroxygruppen als Substituenten von Verbindungen der Formel 1 mit entsprechenden Schutzgruppen versehen sein.

Unter Solvaten der Verbindungen der Formel 1 werden Anlagerungen von inerten Lösungsmittelmolekülen an die Verbindungen der Formel I verstanden, die sich aufgrund ihrer gegenseitigen Anziehungskraft ausbilden. Solvate sind z.B. Mono- oder Dihydrate oder Additionsverbindungen mit Alkoholen, wie z.B. mit Methanol oder Ethanol.

Gegenstand der Erfindung sind die Verbindungen der Formel 1 und ihre Salze und Solvate nach Anspruch 1 sowie ein Verfahren zur Herstellung von Verbindungen der Formel 1 sowie ihrer Salze und Solvate, dadurch gekennzeichnet, daß man
a) eine Verbindung der Formel 1 aus einem ihrer funktionellen Derivate durch Behandeln mit einem solvolysierenden oder hydrogenolysierenden Mittel in Freiheit setzt, oder
b) einen Rest R¹, R², R³, R⁴; R⁵ und/oder R⁶ in einen anderen Rest R¹, R², R³ , R⁴, R⁵ und/oder R⁶ umwandelt, indem man beispielsweise
   i) eine Aminogruppe durch Umsetzung mit einem amidierenden Mittel in eine Guanidinogruppe umwandelt,
   ii) einen Ester verseift,
   iii) eine Aminogruppe alkyliert oder acyliert,
   iv) eine Cyangruppe in eine Amidinogruppe umwandelt,
und/oder eine Base oder Säure der Formel I in eines ihrer Salze umwandelt.

In den vorstehenden Formeln bedeutet Z Alkyl, ist linear oder verzweigt, und hat 1 bis 6, vorzugsweise 1, 2, 3, 4, 5 oder 6 C-Atome. Z bedeutet vorzugsweise Methyl, weiterhin Ethyl, n-Propyl, Isopropyl, n-Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl.
Besonders bevorzugt für Z ist Methyl oder Ethyl.

Ar ist Phenyl, m-Trifluormethoxyphenyl, p-Fluorphenyl, m-Chlorphenyl, m-Hydroxyphenyl, 2,4- oder 3,5-Dichlorphenyl, 4-Chlor-3-trifluormethylphenyl, 2,6-Difluorphenyl, 2,4,6-Trifluorphenyl, 2-Chlor-3,6-difluorphenyl, Ganz besonders bevorzugt ist Ar p-Fluorphenyl.

Hal bedeutet F, Cl, Br oder I, besonders bevorzugt F, Cl oder Br.

Het ist Pyridin-4-yl, oder Benzothiadiazol-5-yl.

Het¹ ist Pyridin-2-yl, Imidazol-2-yl, 4,5-Dihydro-imidazol-2-yl, oder 3,5-Dihydroimidazol-4-on-2-yl.

Die genannten heterocyclischen Ringe können auch ein- oder zweifach durch =O oder NHZ substituiert sein.

A ist NH, CONH, NHCO oder eine direkte Bindung, ganz besonders bevorzugt NH.
B ist O.

X bedeutet eine direkte Bindung.

m bedeutet 3 oder 4. Ganz besonders bevorzugt ist m 3.

n bedeutet 0.

R¹ ist H.

R² ist R² H.

R³ ist 1H-Imidazol-2-yl, 4,5-Dihydro-imidazol-2-yl, 3,5-Dihydro-imidazol-4-on-2-yl oder Pyridin-2-yl.

R⁴ ist Phenyl, 3-Trifluormethoxyphenyl, 4-Fluorphenyl, 3-Chlorphenyl, 3-Hydroxyphenyl, Pyridin-4-yl, 3,5-Dichlorphenyl, 2,4-Dichlorphenyl, Cyclohexyl, 4-Chlor-3-trifluormethylphenyl, Benzothiadiazol-5-yl, 2,6-Difluorphenyl, 2-Chlor-3,6-difluorphenyl, 2,4,6-Trifluorphenyl oder Cyclohexyl. R⁵ ist H.

Bevorzugte Ausführungen für den Substituenten R³-(CH₂)ₙ-A-(CH₂)ₘ-Bsind 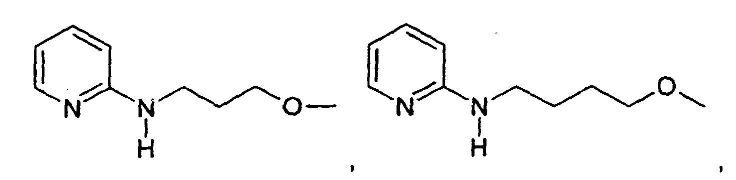 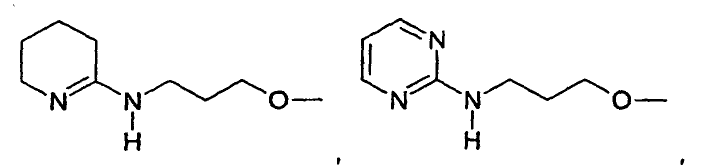 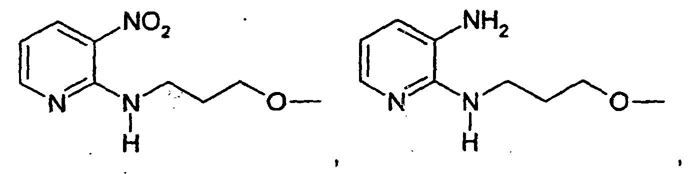 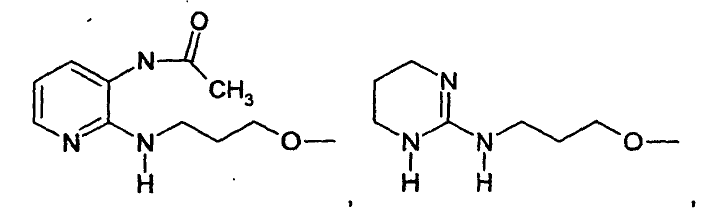 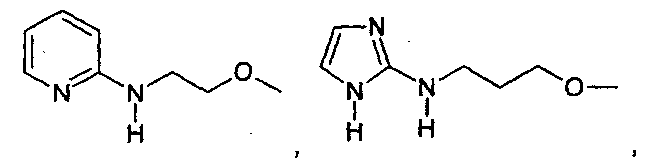 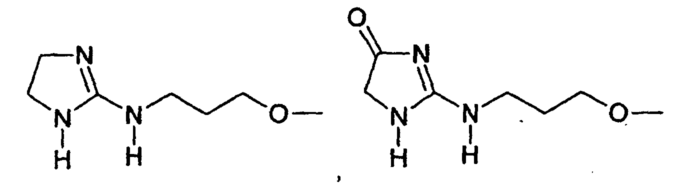 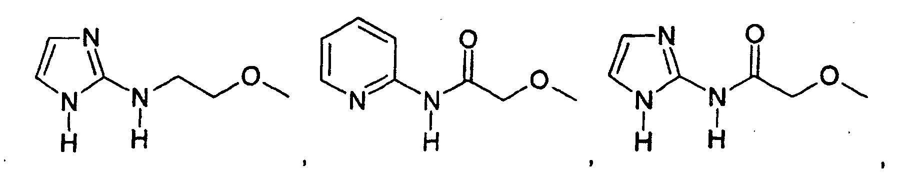 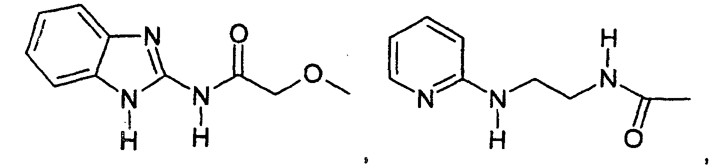 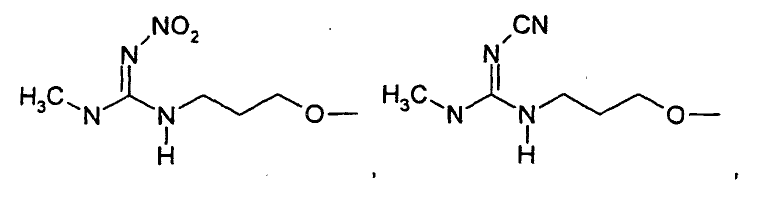 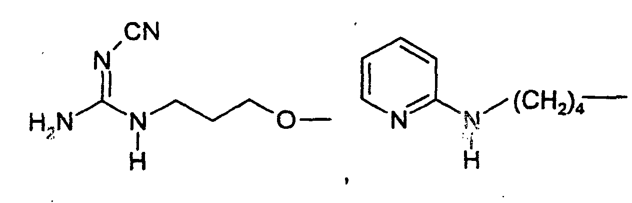 oder

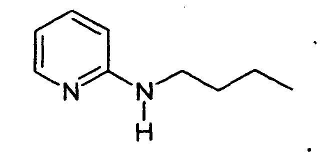

Bevorzugt befindet sich der Substituent R³-(CH₂)ₙ-A-(CH₂)ₘ-B- in der 5-oder 6-Position des Indolrings, besonders bevorzugt in 6-Position.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ii ausgedrückt werden, die der Formel I entsprechen und worin die nicht näher bezeichneten Reste die bei der Formel I angegebene Bedeutung haben, worin jedoch
in Ia
   - X: eine direkte Bindung bedeutet 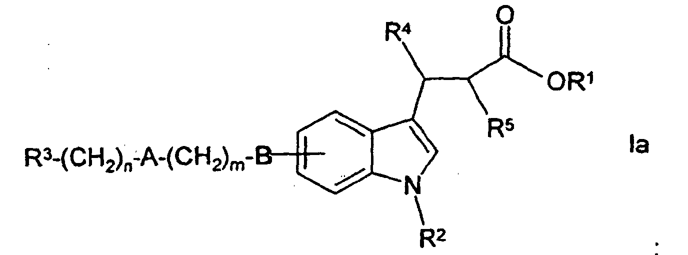
in Ib
   - X: eine direkte Bindung,
   - R²: H,
   - R⁵: H,
   - R⁴: (CH₂)ₒ-Ar und
   - o: 0 bedeutet 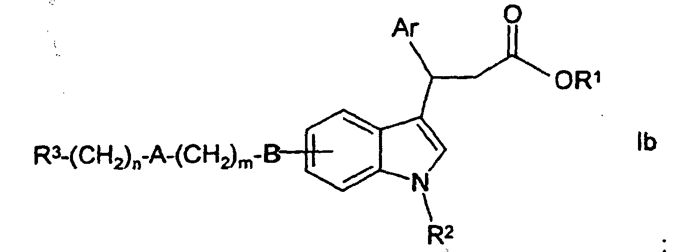
in Ic
   - X: eine direkte Bindung,
   - R⁵: H,
   - R⁴: (CH₂)ₒ-Ar oder Het und
   - o: 0
   bedeutet;
in Id
   - X: eine direkte Bindung,
   - R⁵: H,
   - B: O,
   - A: NH,
   - n: 0,
   - m: 3 oder 4,
   - R³: Het¹,
   - R⁴: (CH₂)ₒ-Ar und
   - o: 0 bedeutet 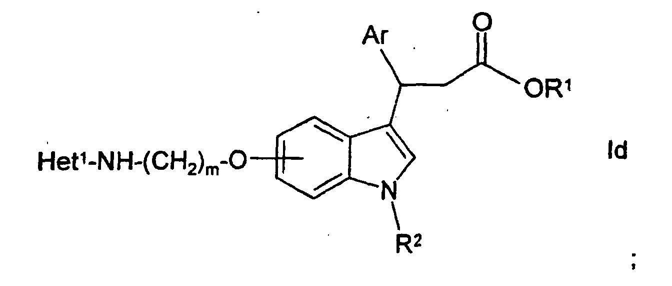
in Ie
   - X: eine direkte Bindung,
   - R⁵: H,
   - B: O,
   - A: NH,
   - n: 0,
   - m: 3 oder 4 und
   - R³: Het¹ bedeutet

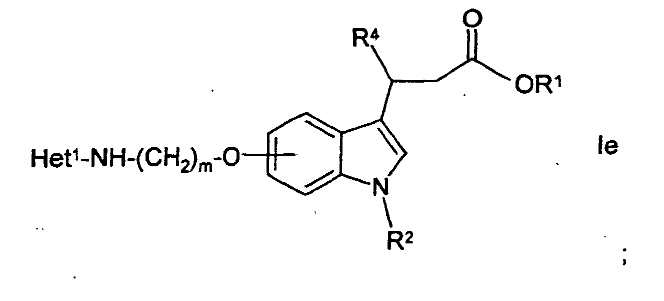

Die Verbindungen der Formel I nach Anspruch 1 und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel 1 nach Anspruch 1 umsetzt.

Verbindungen der Formel I können vorzugsweise erhalten werden, indem man Verbindungen der Formel 1 aus einem ihrer funktionellen Derivate durch Behandeln mit einem solvolysierenden oder hydrogenolysierenden Mittel in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, insbesondere solche, die anstelle einer H-N-Gruppe eine SG¹-N-Gruppe tragen, worin SG¹ eine Aminoschutzgruppe bedeutet und/oder solche, die anstelle des H-Atoms einer Hydroxygruppe eine Hydroxyschutzgruppe tragen, z.B. solche, die der Formel entsprechen, jedoch anstelle einer Gruppe -COOH eine Gruppe -COOSG² tragen, worin SG² eine Hydroxyschutzgruppe bedeutet.

Es können auch mehrere - gleiche oder verschiedene - geschützte Aminound/oder Hydroxygruppen im Molekül des Ausgangsstoffes vorhanden sein. Falls die vorhandenen Schutzgruppen voneinander verschieden sind, können sie in vielen Fällen selektiv abgespalten werden (vgl. dazu: T.W. Greene, P.G.M. Wuts, *Protective Groups in Organic Chemistry*, 2. Aufl., Wiley, New York **1991** oder P.J. Kocienski, *Protecting Groups*, 1. Aufl., Georg Thieme Verlag, Stuttgart - New-York, **1994**, H. Kunz, H. Waldmann in *Comprehensive Organic Synthesis*, Vol. 6 (Hrsg. B.M. Trost, I. Fleming, E. Winterfeldt), Pergamon, Oxford, **1991**, S. 631-701).

Der Ausdruck "Aminoschutzgruppe" ist allgemein bekannt und bezieht sich auf Gruppen, die geeignet sind, eine Aminogruppe vor chemischen Umsetzungen zu schützen (zu blockieren). Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Synthesesequenz) entfernt werden, ist ihre Art und Größe im übrigen nicht kritisch; bevorzugt werden jedoch solche mit 1-20 C-Atomen. Der Ausdruck "Acylgruppe" ist im Zusammenhang mit dem vorliegenden Verfahren im weitesten Sinne aufzufassen. Er umschließt von aliphatischen, araliphatischen, alicyclischen, aromatischen oder heterocyclischen Carbonsäuren oder Sulfonsäuren abgeleitete Acylgruppen sowie insbesondere Alkoxycarbonyl-, Alkenyloxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxy-carbonyl, Boc, 2-Iodethoxycarbonyl; Alkenyloxycarbonyl wie Allyloxycarbonyl (Aloc), Aralkyloxycarbonyl wie CBZ (synonym mit Z), 4-Methoxybenzyloxycarbonyl (MOZ), 4-Nitro-benzyloxycarbonyl oder 9-Fluorenylmethoxycarbonyl (Fmoc); 2-(Phenylsulfonyl)ethoxycarbonyl; Trimethylsilylethoxycarbonyl (Teoc) oder Arylsulfonyl wie 4-Methoxy-2,3,6-trimethylphenyl-sulfonyl (Mtr). Bevorzugte Aminoschutzgruppen sind Boc, Fmoc und Aloc, ferner Z, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen. Typisch für solche Gruppen sind die oben genannten unsubstituierten oder substituierten Aryl-, Aralkyl-, Aroyl- oder Acylgruppen, ferner auch Alkylgruppen, Alkyl-, Aryl- oder Aralkyl-silylgruppen oder O,O- oder O,S-Acetale. Die Natur und Größe der Hydroxyschutzgruppen ist nicht kritisch, da sie nach der gewünschten chemischen Reaktion oder Synthesesequenz wieder entfernt werden; bevorzugt sind Gruppen mit 1-20, insbesondere 1-10 C-Atomen. Beispiele für Hydroxyschutzgruppen sind u.a. Aralkylgruppen wie Benzyl, 4-Methoxybenzyl oder 2,4-Dimethoxybenzyl, Aroylgruppen wie Benzoyl oder p-Nitrobenzoyl, Acylgruppen wie Acetyl oder Pivaloyl, p-Toluolsulfonyl, Alkylgruppen wie Methyl oder tert.-Butyl, aber auch Allyl, Alkylsilylgruppen wie Trimethylsilyl (TMS), Triisopropylsilyl (TIPS), tert.-Butyldimethylsilyl (TBS) oder Triethylsilyl, Trimethylsilylethyl, Aralkylsilylgruppen wie tert.-Butyldiphenylsilyl (TBDPS), cyclische Acetale wie Isopropyliden-, Cyclopentyliden-, Cyclohexyliden-, Benzyliden-, p-Methoxybenzyliden- oder o,p-Dimethoxybenzylidenacetal, acyclische Acetale wie Tetrahydropyranyl (Thp), Methoxymethyl (MOM), Methoxyethoxymethyl (MEM), Benzyloxymethyl (BOM) oder Methylthiomethyl (MTM). Besonders bevorzugte Hydroxyschutzgruppen sind Benzyl, Acetyl, tert.-Butyl oder TBS.

Das In-Freiheit-Setzen der Verbindungen der Formel I aus ihren funktionellen Derivaten ist für die jeweils benutzte Schutzgruppe aus der Literatur bekannt (z.B. T.W. Greene, P.G.M. Wuts, *Protective Groups in Organic Chemistry*, 2. Aufl., Wiley, New York **1991** oder P.J. Kocienski, *Protecting Groups*, 1. Aufl., Georg Thieme Verlag, Stuttgart - New-York, **1994**). Dabei kann man auch von an sich bekannten, hier nicht näher erwähnten Varianten Gebrauch machen.

Verbindungen der Formel I, in denen R³ = Het¹, B = O, A = NH und n = 0 bedeutet (Formel I-1), können vorzugsweise nach folgendem Reaktionsschema 1 erhalten werden. SG³ oder SG⁴ sind Hydroxyschutzgruppen, wie zuvor definiert. SG⁵ ist eine Aminoschutzgruppe, wie zuvor beschrieben. Die in den Verbindungen **I-1, II-VI** genannten Reste X, R¹, R², R⁴ und R⁵ und die Variable m haben die in Anspruch 1 angegebenen Bedeutungen.

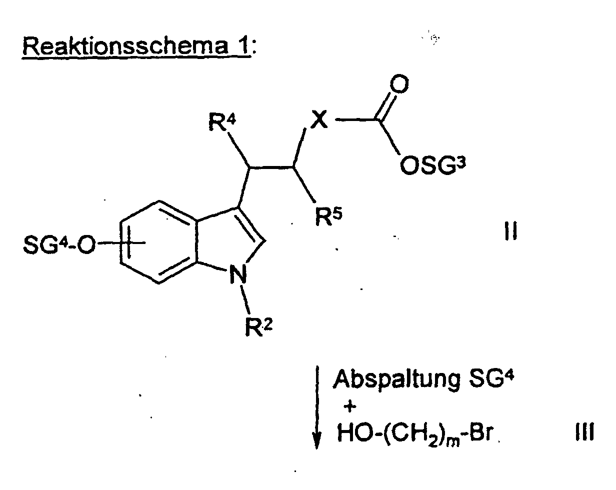

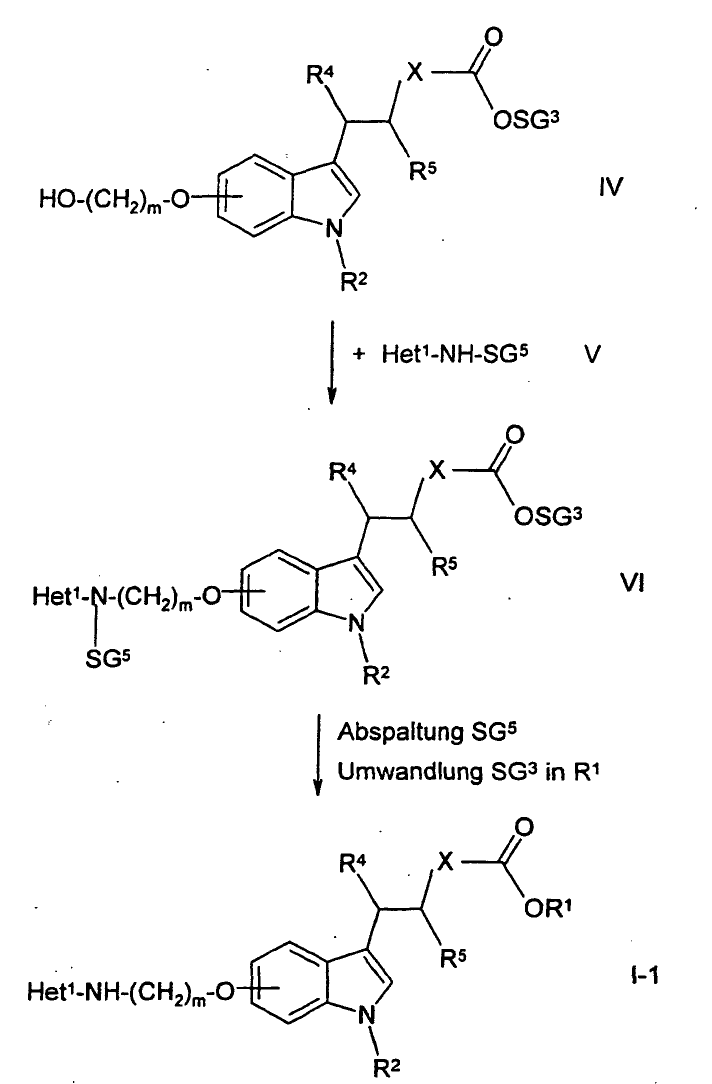

Nach Abspaltung der Hydroxyschutzgruppe SG⁴ der Verbindung der Formel II unter den entsprechenden bekannten Reaktionsbedingungen wird analog zu Reaktionsbedingungen von nucleophilen Substitutionen mit der Verbindung der Formel III umgesetzt. Unter den bekannten Reaktionsbedingungen für eine Mitsunobu Reaktion [Literatur. O.

Mitsunobu, Synthesis 1981, 1-28] wird im nachfolgenden Schritt mit einer Verbindung der Formel V umgesetzt und die Aminoschutzgruppe SG⁵ entsprechend deblockiert. Eine Abspaltung der Hydroxyschutzgruppe SG³ führt zu einer freien Säure der Formel I-1 (R¹ = H). Gegebenenfalls wird die Hydroxyschutzgruppe SG³ in einen Substitutenten R¹ umgewandelt.

Gegenstand der Erfindung sind ebenfalls Verbindungen der Formel IIa 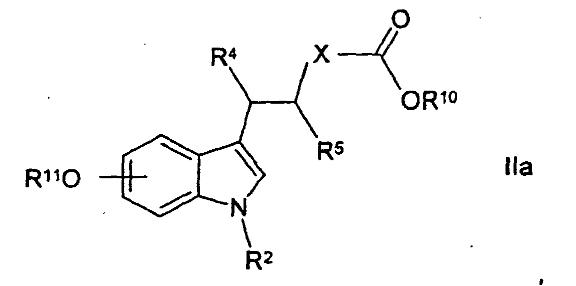 worin R², R⁴ oder R⁵ eine in Anspruch 1 angegebene Bedeutung haben und
- X: eine Bindung,
- R¹⁰: eine Hydroxyschutzgruppe oder H und
- R¹¹: eine Hydroxyschutzgruppe oder H bedeuten.

Bevorzugt ist R¹⁰ H oder eine Alkylgruppe Z als Hydroxyschutzgruppe, wobei Z eine der zuvor beschriebenen Bedeutungen hat.
Bevorzugt ist R¹¹ H oder eine Aralkylgruppe als Hydroxyschutzgruppe, wie zuvor beschrieben.
Bevorzugt steht die Hydroxygruppe OR¹¹ in 6-Position des Indol-Ringes. Verbindungen der Formel IIa sind wertvolle Zwischenstufen bei der Synthese der erfindungsgemäßen Verbindungen der Formel I, worin X eine Bindung bedeutet.

Bevorzugte Verbindungen der Formel IIa sind
3-Phenyl-3-(6-O-benzyl-indol-3-yl)-propionsäure-ethylester;
3-Phenyl-3-(6-hydroxy-indol-3-yl)-propionsäure-ethylester;
3-Phenyl-3-(5-O-benzyl-indol-3-yl)-propionsäure-ethylester;
3-(3-Chlor-phenyl)-3-(6-O-benzyl-indol-3-yl)-propionsäure-ethylester;
3-(3-Chlor-phenyl)-3-(6-hydroxy-indol-3-yl)-propionsäure-ethylester;
3-(6-Benzyloxy-1 H-indol-3-yl)-3-pyridin-4-yl-propionsäure-ethylester;
3-(6-Hydroxy-1 H-indol-3-yl)-3-pyridin-4-yl-propionsäure-ethylester;
3-Benzo[1,2,5]thiadiazol-5-yl-3-(6-benzyloxy-1H-indol-3-yl)-propionsäureethylester;
3-Benzo[1,2,5]thiadiazol-5-yl-3-(6-hydroxy-1H-indol-3-yl)-propionsäureethylester;
3-Cyclohexyl-3-(6-benzyloxy-1H-indol-3-yl)-propionsäure-ethylester;
3-Cyclohexyl-3-(6-hydroxy-1H-indol-3-yl)-propionsäure-ethylester; Verbindungen der Formel IIa, wie zuvor definiert, können analog zu Beispiel 1 nach dem folgenden Reaktionsschema 1a hergestellt werden, wobei R⁵ H und R¹¹ eine Hydroxyschutzgruppe SG⁴ bedeutet.

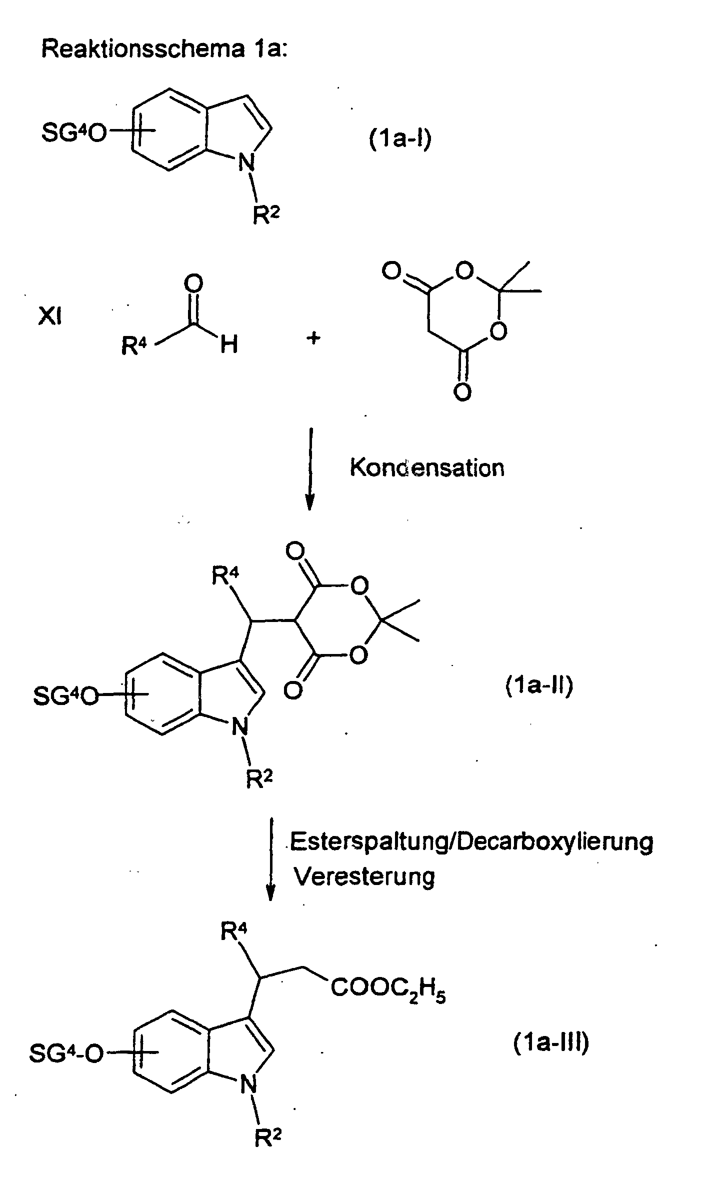

Die Kondensation einer Verbindung der Formel (1a-I) mit einem Aldehyd XI und 2,2-Dimethyl-[1,3]dioxan-4,6-dion (Meldrumsäure) führt unter für Kondensationsreaktionen bekannten Reaktionsbedingungen zu Verbindungen der Formel (1a-II). Nach kombinierter Esterpaltung/Decarboxylierung/Veresterung wird der Ethylester der Formel (1a-III) erhalten. Die Hydroxyschutzgruppe SG⁴ kann nach literaturbekannten Verfahren entfernt werden und man erhält die freien Hydroxyverbindungen der Formel IIa. Eine Esterspaltung der Verbindungen der Formel (1a-II) oder den Hydroxy-Analogen führt zu freien Säuren der Formel IIa.

Verbindungen der Formel I, in denen R³ = Het¹, B = O, A = NHCO und n = 0 bedeutet (Formel I-2), können vorzugsweise nach folgendem Reaktionsschema 2 erhalten werden. SG³, SG⁴ oder SG⁶ sind Hydroxyschutzgruppen, wie zuvor definiert. Die in den Verbindungen I-2, II, VII bis IX genannten Reste X, R¹, R², R⁴ und R⁵ und die Variable m haben die in Anspruch 1 angegebenen Bedeutungen.

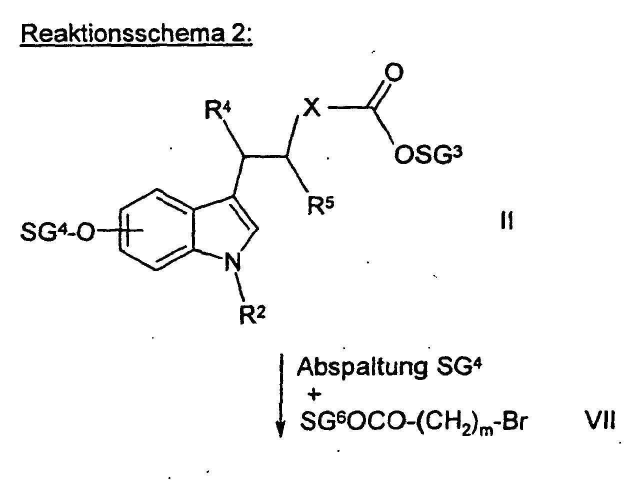

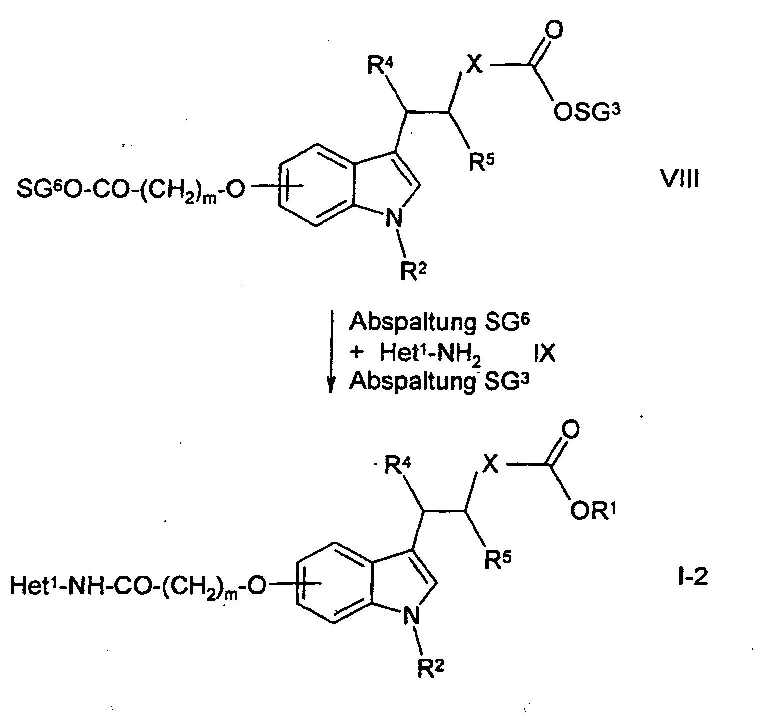

Nach Abspaltung der Hydroxyschutzgruppe SG⁴ der Verbindung der Formel II unter den entsprechenden bekannten Reaktionsbedingungen wird analog zu Reaktionsbedingungen von nucleophilen Substitutionen mit der Verbindung der Formel VII umgesetzt. Nach Abspaltung der Hydroxyschutzgruppe SG⁶ wird unter den bekannten Reaktionsbedingungen für peptidanaloge Kupplungen mit einer Verbindung der Formel IX umgesetzt. Eine Abspaltung der Hydroxyschutzgruppe SG³ führt zu einer freien Säure der Formel I-2 (R¹ = H). Gegebenenfalls wird die Hydroxyschutzgruppe SG³ in einen Substitutenten R¹ umgewandelt.

Verbindungen der Formel I, in denen B = O, X = eine Bindung, R¹ = H und R⁵ = H bedeutet (Formel I-3), können vorzugsweise nach folgendem Reaktionsschema 3 erhalten werden. Die in den Verbindungen X-XII genannten Reste R³, R² und R⁴ und die Variablen A, n und m haben die in Anspruch 1 angegebenen Bedeutungen, wobei während der Synthese freie Aminogruppen in R³ durch Aminoschutzgruppen geschützt vorliegen und im letzten Reaktionsschritt die Schutzgruppen abgespaltet werden.

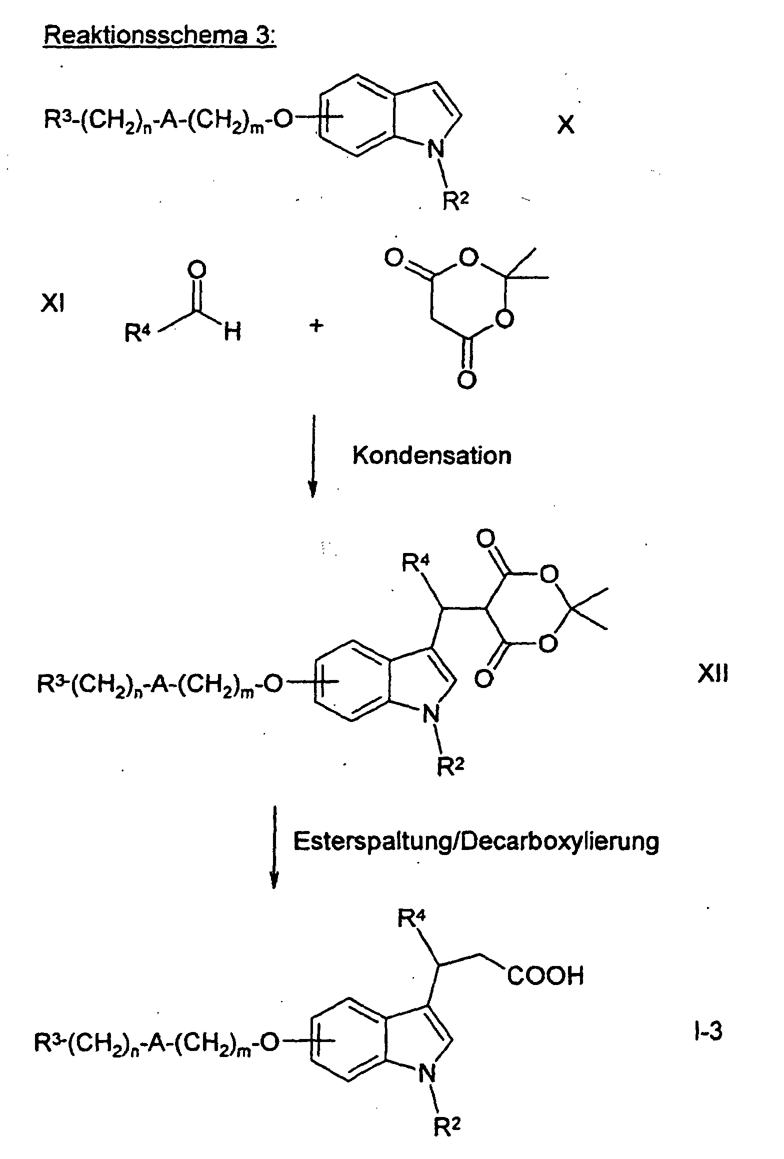

Die Kondensation einer Verbindung der Formel X mit einem Aldehyd XI und 2,2-Dimethyl-[1,3]dioxan-4,6-dion führt unter für Kondensationsreaktionen bekannten Reaktionsbedingungen zu Verbindungen der Formel XII. Nach Esterspaltung und Decarboxylierung wird die freie Säure der Formel I-3 erhalten. Gegebenenfalls wird die Hydroxygruppe in einen Substitutenten R¹ oder die Säure der Formel I-3 in ein physiologisch unbedenkliches Salz umgewandelt.
Verbindungen der Formel X erhält man durch Alkylierung von 1 H-Indol-6-ol mit einem Bromid der Formel XIII (R³-(CH₂)ₙ-A-(CH₂)ₘ-Br XIII), worin der genannte Rest R³ und die Variablen A, n und m haben die in Anspruch 1 angegebenen Bedeutungen haben.

Verbindungen der Formel I, in denen R³ = Het¹, R⁵ = H, X = eine Bindung, A = NH, B = O und n = 0 bedeutet (Formel I-4), können vorzugsweise nach folgendem Reaktionsschema 4 erhalten werden. In den Verbindungen der Formel IIa, wie zuvor beschrieben, bedeuten R¹⁰ SG³ und R¹¹ SG⁴ (Formel IIa-1), wobei SG³ und SG⁴ Hydroxyschutzgruppen sind, wie zuvor definiert. SG⁵ ist eine Aminoschutzgruppe, wie zuvor beschrieben. Die in den Verbindungen I-4, XV-XVIII genannten Reste R¹, R² und R⁴ und die Variable m haben die in Anspruch 1 angegebenen Bedeutungen.

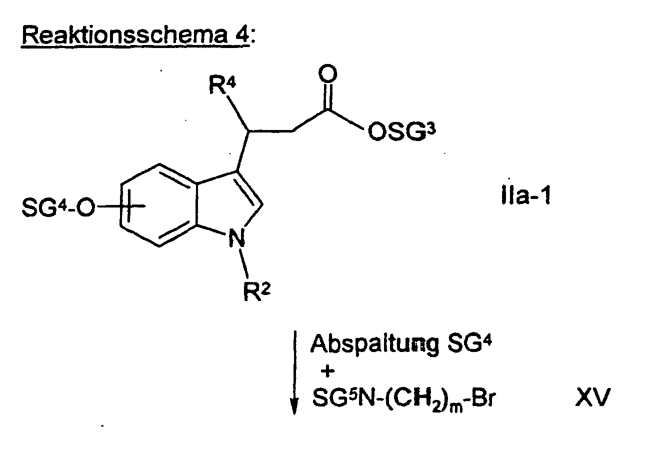

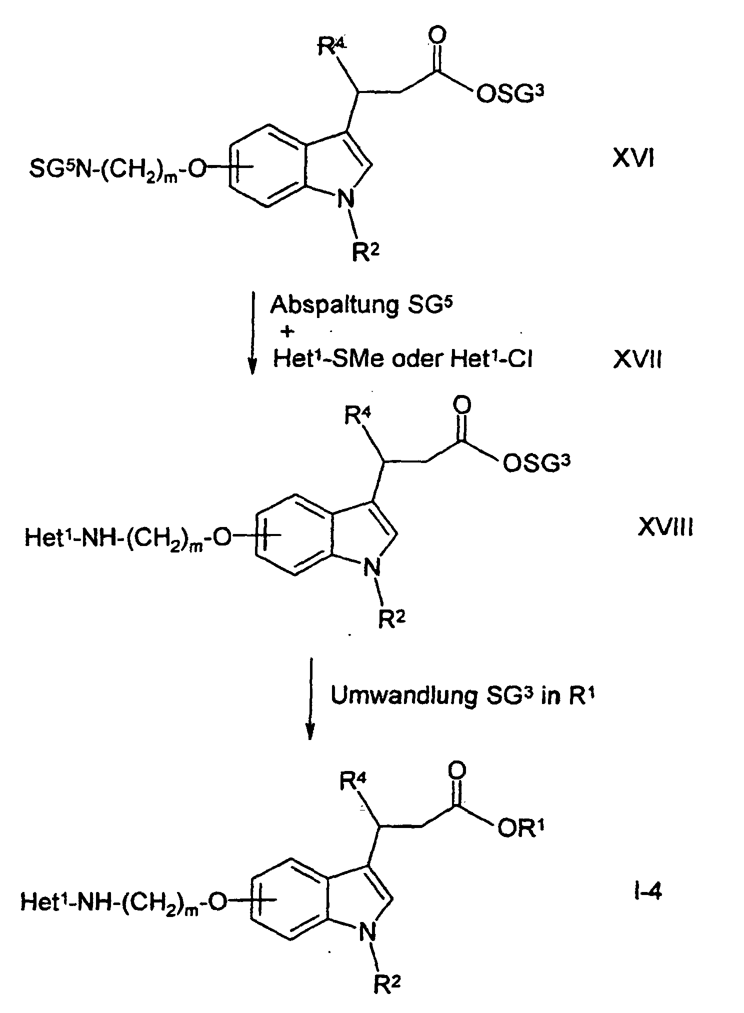

Nach Abspaltung der Hydroxyschutzgruppe SG⁴ der Verbindung der Formel IIa-1 des Reaktionsschematas 4 unter den entsprechenden bekannten Reaktionsbedingungen, wird analog zu Reaktionsbedingungen von nucleophilen Substitutionen mit der Verbindung der Formel XV umgesetzt. Im nachfolgenden Schritt wird die Aminoschutzgruppe SG⁵ abgespalten und das freie Amin mit einer Thiomethyl- oder Chlor-Verbindung der Formel XVII umgesetzt. Eine Abspaltung der

Hydroxyschutzgsuppe SG³ führt zu einer freien Säure der Formel 1-4 (R¹ = H). Gegebenenfalls wird die Hydroxyschutzgruppe SG³ in einen Substitutenten R¹ umgewandelt.

Verbindungen der Formel X 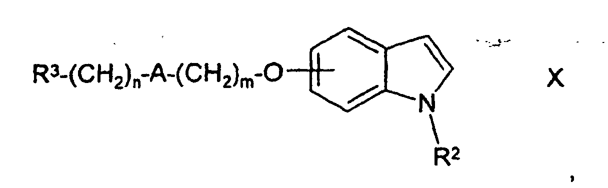 worin R², R³, A, n und m eine in Anspruch 1 angegebene Bedeutung haben, können analog der Synthesesequenz des Reaktionsschematas 4 hergestellt werden, indem anstelle der Verbindung IIa-1 eine hydroxysubstituierte indolverbindung XXII 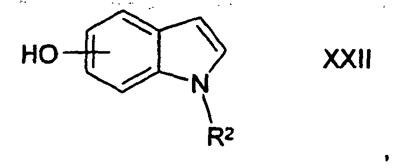 worin R² eine in Anspruch 1 angegebene Bedeutung hat, eingesetzt wird. Nach Umsetzung des Hydroxyindols XXII mit einer Verbindung der Formel XV und Abspaltung der Aminoschutzgruppe SG⁵, wie zuvor beschrieben, kann, je nach Substituent R³ mit einer Verbindung der Formel XVII oder XIX umgesetzt werden oder mit einer Verbindung der Formel XX und sich anschließender Reaktion mit einer Verbindung der Formel XXI. Freie Aminogruppen in Verbindungen der Formel XVII sind während der Synthese durch Aminoschutzgruppen geschützt.

Gegenstand der Erfindung sind ebenfalls Verbindungen der Formel X 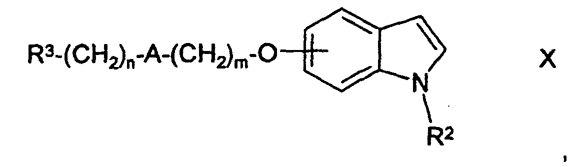 worin
R², R³, A, n und m eine in Anspruch 1 angegebene Bedeutung haben oder deren Salze.

Bevorzugte Verbindungen der Formel X sind
6-(3-(Pyridin-2-yl-amino)-propyloxy)-indol;
6-[3-(4,5-Dihydro-1 H-imidazol-2-yl-amino)-propyloxy]-indol oder
6-[3-(4,5-Dihydro-1 H-imidazol-2-yl-amino)-butyloxy]-indol.

Als inerte Lösungsmittel eignen sich z.B. Kohlenwasserstoffe wie Hexan, Petrolether, Benzol, Toluol oder Xylol; chlorierte Kohlenwassertoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkchlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Ferner ist es möglich, daß man einen Rest R¹, R², R³, R⁴, R⁵ und/oder R⁶ in einen anderen Rest R¹, R², R³ , R⁴, R⁵ und/oder R⁶ umwandelt.
So ist es möglich, einen Ester der Formel 1 unter Standardbedingungen, z.B. NaOH in Dioxan/Wasser, 0-60°C, zu verseifen.
Die Umwandlung einer Cyangruppe in eine Amidinogruppe erfolgt durch Umsetzung mit z.B. Hydroxylamin und anschließender Reduktion des N-Hydroxyamidins mit Wasserstoff in Anwesenheit eines Katalysators wie z.B. Pd/C.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und/oder in Gegenwart einer Base wie Triethylamin oder Pyridin bei Temperaturen zwischen -60 und +30°C.

Eine Base der Formel I kann mit einer Säure in das zugehörige Säureadditionssalz überführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, schweflige Säure, Dithionsäure, Salpetersäure, Halogenwasserstoffsäuren wie Chlorwasserstoffsäure oder Bromwasserstoffsäure, Phosphorsäuren wie z.B. Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Hexansäure, Octansäure, Decansäure, Hexadecansäure, Octadecansäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Benzolsulfonsäure, Trimethoxybenzoesäure, Adamantancarbonsäure, p-Toluolsulfonsäure, Glycolsäure, Embonsäure, Chlorphenoxyessigsäure, Asparaginsäure, Glutaminsäure, Prolin, Glyoxylsäure, Palmitinsäure, Parachlorphenoxyisobuttersäure, Cyclohexancarbonsäure, Glucose-1-phosphat, Naphthalin-mono- und disulfonsäuren oder Laurylschwefelsäure. Salze mit physiologisch nicht unbedenklichen Säuren, z.B. Pikrate, können zur Isolierung und/oder Aufreinigung der Verbindungen der Formel I verwendet werden. Andererseits können Verbindungen der Formel 1 mit Basen (z.B. Natriumoder Kaliumhydroxid oder -carbonat) in die entsprechenden Metall-, insbesondere Alkalimetall- oder Erdalkalimetall- oder in die entsprechenden Ammoniumsalze umgewandelt werden. Als Salze kommen ferner substituierte Ammoniumsalze, z.B. die Dimethyl-, Diethyloder Diisopropyl-ammoniumsalze, Monoethanol-, Diethanoi- oder Diisopropylammoniumsalze, Cyclohexyl-, Dicyclohexylammoniumsalze, Dibenzylethylendiammoniumsalze, weiterhin z.B. Salze mit Arginin oder Lysin.

Die Verbindungen der Formel I enthalten mindestens ein chirales Zentrum und können daher in racemischer oder in optisch-aktiver Form vorliegen. .Erhaltene Racemate können nach an sich bekannten Methoden mechanisch oder chemisch in die Isomeren getrennt werden. Vorzugsweise werden aus dem racemischen Gemisch durch Umsetzung mit einem optisch aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch aktiven Camphersulfonsäuren wie β-Camphersulfonsäure. Vorteilhaft ist auch eine Enantiomerentrennung mit Hilfe einer mit einem optisch aktiven Trennmittel (z.B. Dinitrobenzoyl-phenylglycin) gefüllten Säule; als Laufmittel eignet sich z.B. ein Gemisch Hexan/Isopropanol/Acetonitril, z.B. im Volumenverhältnis 82:15:3.
Eine Diastereomerentrennung kann auch durch Standardreinigungsprozesse, wie z.B. Chromatographie oder fraktionierte Kristallisation erfolgen.

Natürlich ist es auch möglich, optisch aktive Verbindungen der Formel 1 nach den oben beschriebenen Methoden zu erhalten, indem man Ausgangsstoffe verwendet, die bereits optisch aktiv sind.

Gegenstand der Erfindung sind ferner pharmazeutische Zubereitungen, enthaltend mindestens eine Verbindung der Formel 1 und/oder eines ihrer physiologisch unbedenklichen Salze oder Solvate, die insbesondere auf nicht-chemischem Wege hergestellt werden. Hierbei können die Verbindungen der Formel 1 zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlenhydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacksund/oder mehrere weitere Wirkstoffe enthalten, z.B. ein oder mehrere Vitamine.

Für die Applikation als Inhalationsspray können Sprays verwendet werden, die den Wirkstoff entweder gelöst oder suspendiert in einem Treibgas oder Treibgasgemisch (z.B. CO₂ oder Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher lnhaiatoren verabreicht werden.

Die Verbindungen der Formel I und ihre physiologisch unbedenklichen Salze können als Integrininhibitoren bei der Bekämpfung von Krankheiten, insbesondere von Thrombosen, Herzinfarkt, koronaren Herzerkrankungen, Arteriosklerose, Tumoren, Osteoporose, Entzündungen und Infektionen verwendet werden.

Die Verbindungen der Formel 1 nach Anspruch 1 und/oder ihre physiologisch unbedenklichen Salze finden auch Verwendung bei pathologischen Vorgängen, die durch Angiogenese unterhalten oder propagiert werden, insbesondere bei Tumoren, Restenosen, diabetischer Retinopathie, makularer degenerativer Krankheit oder rheumatoider Arthritis.

Dabei werden die erfindungsgemäßen Substanzen in der Regel in Analogie zu anderen bekannten, im Handel befindlichen Peptiden, insbesondere aber in Analogie zu den in WO 99/30713 und WO 94/12478 beschriebenen Verbindungen verabreicht, vorzugsweise in Dosierungen zwischen etwa 0,05 und 500 mg, insbesondere zwischen 0,5 und 100 mg pro Dosierungseinheit. Die tägliche Dosierung liegt vorzugsweise zwischen etwa 0,01 und 2 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Vor- und nachstehend sind alle Temperaturen in °C angegeben. In den nachfolgenden Beispielen bedeutet "übliche Aufarbeitung": Man wäscht die organische Phase mit gesättigter NaHCO₃-Lösung, gegebenenfalls mit Wasser und gesättigter NaCl-Lösung, trennt ab, trocknet die organische Phase über Natriumsulfat, dampft ein und reinigt duch Chromatographie an Kieselgel, durch präparative HPLC und/oder durch Kristallisation. Die gereinigten Verbindungen werden gegebenenfalls gefriergetrocknet.

HPLC: Eluent A = Wasser + 0.3% TFA, Eluent B = Acetonitil/Wasser + 0.3% TFA im Verhältnis 4:1. Rₜ bedeutet Retentionszeit. R_{f} bedeutet Retentionsfaktor.

### Beispiel 1:

### 1. 5-[Phenyl-(6-O-benzyl-indol-3-yl)-methyl]-2,2-dimethyl-[1,3]dioxan-4,6-dion 2

5 g (22.4 mmol) 6-Benzytoxy-indol werden mit 2.26 ml (22.4 mmol) Benzaldehyd und 3.23 g (22.4 mmol) Meldrumsäure (2,2-Dimethyl-[1,3]dioxan-4,6-dion) in 100 ml wasserfreiem Acetonitril gelöst und in Gegenwart von 129 mg (1.1 mmol) L-Prolin bei 30°C bis zur vollständigen Umsetzung gerührt (3 h, DC-Kontrolle). Man läßt auf Raumtemperatur abkühlen, saugt den entstandenen Niederschlag ab und wäscht diesen mit Ether aus. Nach gutem Trocknen wird das Rohprodukt 5-[Phenyl-(6-Obenzyl-indol-3-yl)-methyl]-2,2-dirnethyl-[1,3]dioxan-4,6-dion ohne weitere Reinigung weiter umgesetzt.

HPLC: (RP-18, Gradient A/B 50:50→1:99 in 1 h mit A = Wasser + 0.3%TFA, B = Acetonitil/Wasser + 0.3% TFA 4:1) Rₜ = 41.4 min;
DC: Si-60, Toluol/Aceton 4:1, R_{f} = 0.3;
FAB-MS: (M+1) = 456.

### 2. 3-Phenyl-3-(6-O-benzyl-indol-3-yl)-propionsäure-ethylester 3

5 g (11 mmol) **2** werden mit 300 mg Kupferpulver und 3 ml getrocknetem Ethanol in 30 ml wassserfreiem Pyridin vorgelegt und 3 h unter Rückfluß und Rühren gekocht (DC-Kontrolle). Anschließend filtriert man über Kieselgur, engt die Lösung ein und nimmt den Rückstand in Ethylacetat auf. Es wird wie üblich aufgearbeitet. Man erhält 3-Phenyl-3-(6-O-benzylindol-3-yl)-propionsäure-ethylester, der durch Chromatographie an Kieselgel mit Toluol/Aceton 20:1 als Eluent gereinigt wird.

HPLC: (RP-18, Gradient A/B 50:50→1:99 in 1 h wie oben) Rₜ = 54 min;
DC: Si-60, Toluol/Aceton 4:1, R_{f} = 0.7;
FAB-MS: (M+1) = 400.

### 3. 3-Phenyl-3-(6-hydroxy-indol-3-yl)-propionsäure-ethylester 4

3.7 g (9.26 mmol) **3** werden in 60 ml Ethanol gelöst und bei Raumtemperatur und Normaldruck 2.5 h in Gegenwart von 900 mg Palladium/10% auf Aktivkohle hydriert. Nach vollständiger Benzylspaltung filtriert man vom Katalysator ab, wäscht mit etwas Ethanol nach und engt die Lösung ein. Man erhält 3-Phenyl-3-(6-hydroxy-indol-3-yl)-propionsäureethylester.

HPLC: (RP-18, GradientAlB 99:1→1:99 in 1 h) Rₜ =40.3 min;
DC: Si-60, Toluol/Aceton 4:1, R_{f} = 0.2;
FAB-MS: (M+1 ) = 310.

### 4. 3-Phenyl-3-[6-(3-hydroxy-propyloxy)-indol-3-yl]-propionsäure-ethylester 5

1.2 g (3.88 mmol) **4** werden mit 0.66 ml (7.6 mmol) 3-Brom-1-propanol und 2.1 g (15.2 mmol) Kaliumcarbonat in 30 ml Aceton über Nacht unter Rückfluß gekocht. Nach dem Abkühlen filtriert man vom unlöslichen Rückstand und engt das Filtrat ein. Das Rohprodukt kann durch Chromatographie an Kieselgel (Eluentengradient Toluol/Aceton 9:1→4:1) gereinigt werden. Man erhält 3-Phenyt-3-[6-(3-hydroxy-propyfoxy)-indol-3-yl]-propionsäure-ethylester.

HPLC: (RP-18, Gradient A/B 99:1→1:99 in 1 h) Rₜ =42.4 min;
DC: Si-60, Toluol/Aceton 4:1, R_{f} = 0.1;
FAB-MS: (M+1) = 368.

### 5. 3-Phenyl-3-(6-{3-[(pyridin-2-yl)-(2,2,2-trichlorethoxycarbonyl)-amino]-propyloxy}-indol-3-yl)-propionsäure-ethylester 6

In 7.5 ml wasserfreiem THF werden 500 mg (1.36 mmol) **5** und 550 mg (2.04 mmol) 2-(2,2,2-Trichlorethoxycarbonyl-amino)-pyridin und 907 mg (2.72 mmol) Triphenylphosphin (polymergebunden) vorgelegt und dazu bei Raumtemperatur eine Lösung von 0.32 ml (2.04 mmol) Azodicarbonsäurediethylester (Diethylazodicarboxylat, DEAD) in 7.5 ml THF innerhalb von 30 min zugetropft. Die DC-Kontrolle zeigt nach 1.5 h einen vollständigen Umsatz. Das Polymer wird abfiltriert, die Lösung mit etwas Wasser gewaschen, getrocknet und eingeengt. Der Rückstand läßt sich durch Chromatographie an Kieselgel (Eluentengradient Toluol/Aceton 20:1→4:1). Man erhält 3-Phenyl-3-(6-{3-[(pyridin-2-yl)-(2,2,2-trichlorethoxycarbonyl)-amino]-propyloxy}-indol-3-yl)-propionsäureethylester.

HPLC: (RP-18, Gradient A/B 99:1→1:99 in 1 h) Rₜ = 56.1 min;
DC: Si-60, Toluol/Aceton 4:1, R_{f} = 0.5;
FAB-MS: (M+1) = 619.

### 6. 3-Phenyl-3-{6-[(3-pyridin-2-yl-amino)-propyloxy]-indol-3-yl}-propionsäureethylester 7

275 mg (0.44 mmol) **6** werden mit 500 mg Zinkstaub, 0.5 ml Wasser und 0.5 ml Essigsäure in 5 ml THF bei Raumtemperatur 2.5 h gerührt. Nach vollständiger Reaktion filtriert man vom Zink ab, engt die Lösung ein und reinigt den Rückstand durch präparative HPLC an RP-18 (Eluentengradient Wasser/Acetonitril 99:1→1:99). Man erhält 3-Phenyl-3-{6-[(3-pyridin-2-ylamino}-propyloxy]-indol-3-yl}-propionsäure-ethylester Trifluoracetat.

HPLC: (RP-18, Gradient A/B 99:1→1:99 in 1 h) Rₜ = 42.8 min;
FAB-MS: (M+1 ) = 444.

### 7. 3-Phenyl-3-{6-[3-(pyridin-2-yl-amino)-propyloxy]-indol-3-yl}propionsäure 8

80 mg (0.18 mmol) **7** werden in 2 ml Dioxan gelöst und bei Raumtemperatur über Nacht mit 0.9 ml 1 N NaOH (0.9 mmol) gerührt. Nach vollständiger Esterspaltung neutralisiert man die Lösung mit etwas Essigsäure. Man erhält 3-Phenyl-3-{6-[3-(pyridin-2-yl-amino)-propyloxy]-indol-3-yl}-propionsäure. Nach präparativer HPLC erhält man 3-Phenyl-3-{6-[3-(pyridin-2-yl-amino)-propyloxy]-indol-3-yl}-propionsäure Trifluoracetat; Smp. 232° (Zers.).

HPLC: (RP-18, Gradient A/B 99:1→1:99 in 1 h) Rₜ = 34.7 min;
FAB-MS: (M+1) = 416.

### Beispiel 2:

### 1. 3-Phenyl-3-(6-{3-[(imidazol-2-yl)-(2,2,2-trichlorethoxycarbonyl)-amino]-propyloxy}-indol-3-yl)-propionsäure-ethylester 9

Entsprechend Beispiel 1.5 werden zu einer Lösung von 500 mg (1.36 mmol) **5**, 527 mg (2.04 mmol) ) und 2-(2,2,2-Trichtorethoxycarbonylamino)-imidazol in 7.5 ml wasserfreiem THF 907 mg (2.72 mmol) Triphenylphosphin (polymergebunden) zugegeben und anschließend bei Raumtemperatur 0.32 ml (2.04 mmol) DEAD langsam zugetropft. Man rührt die Lösung über Nacht, filtriert vom Polymer ab, wäscht die THF-Lösung mit Wasser und engt nach dem Trocknen über MgSO₄ ein. Das-Rohprodukt wird durch präparative HPLC gereingt. Man erhält 3-Phenyl-3-(6-{3-[(imidazol-2-yl)-(2,2,2-trichlorethooycarbonyl)-amino]-propyloxy}-indol-3-yl)-propionsäure-ethylester Trifluoracetat.

HPLC: (RP-18, Gradient A/B 99:1→1:99 in 1 h) Rₜ = 47.5 min;
FAB-MS: (M+1 ) = 608.

### 2. 3-Phenyl-3-{6-[3-(imidazol-2-yl-amino)-propyloxy]-indol-3-yl}propionsäure-ethylester 10

Entsprechend Beispiel 1.6 werden 185 mg (0.304 mmol) **9** mit 400 mg Zinkstaub und 0.4 ml Essigsäure in 4 ml THF umgesetzt und aufgearbeitet. Die Reinigung erfolgt durch präparative HPLC an RP-18. Man erhält 3-Phenyl-3-{6-[3-(imidazol-2-yl-amino)-propyloxy]-indol-3-yl}-propionsäureethylester Trifluoracetat.

HPLC: (RP-18, Gradient A/B 99:1→1:99 in 1 h) Rₜ = 40.9 min;
FAB-MS: (M+1) = 433.

### 3. 3-Phenyl-3-{6-[3-(imidazol-2-yl-amino)-propyloxy]-indol-3-yl}propionsäure 11

25 mg (0.058 mmol) **10** werden in 1 ml Dioxan mit 0.3 ml 1 N HCl (0.3 mmol) 36 h bei 70°C gerührt. Man erhält 3-Phenyl-3-{6-[3-(imidazol-2-ylamino)-propyloxy]-indol-3-yl}-propionsäure. Nach präparativer HPLC erhält man 3-Phenyl-3-{6-[3-(imidazol-2-yl-amino)-propyloxy]-indol-3-yl}propionsäure Trifluoracetat.

HPLC: (RP-18, Gradient A/B 99:1→1:99 in 1 h) Rₜ = 33.4 min;
FAB-MS: (M+1) = 405.

### Beispiel 3:

Analog zu Beispiel 1 erhält man aus der Umsetzung von 6-Benzyloxy-indol mit 3-Chlor-benzaldehyd und anschließender Synthesesequenz
3-(3-Chlor-phenyl-3-{6-[3-(pyridin-2-yl-amino)-propyloxy]-indol-3-yl}propionsäure. Nach präparativer HPLC: 3-(3-Chlor-phenyl)-3-{6-[3-(pyridin-2-yl-amino)-propyloxy]-indol-3-yl}-propionsäure Trifluoracetat;
HPLC: (RP-18, Gradient A/B 99:1→1:99 in 1 h) Rₜ = 34.3 min;
FAB-MS: (M+1) = 450;
mit Pyridin-4-carbaldehyd und anschließender Synthesesequenz
3-Pyridin-4-yl-3-{6-[3-(pyridin-2-ylamino)-propoxy]-1H-indol-3-yl}propionsäure. Nach präparativer HPLC: 3-Pyridin-4-yl-3-{6-[3-(pyridin-2-ylamino)-propoxy]-1H-indol-3-yl}-propionsäure Trifluoracetat;
HPLC: (RP-18, Gradient A/B 99:1→1:99 in 1 h) Rₜ = 20.7 min;
FAB-MS: (M+1) = 417;

### Beispiel 4:

Analog zu Beispiel 2 erhält man aus der Umsetzung von 6-Benzyloxy-indol
mit 4-Fluor-benzaldehyd und anschließender Synthesesequenz
3-(4-Fluor-phenyl-3-{6-[3-(imidazol-2-yl-amino)-propyloxy]-indol-3-yl}propionsäure. Nach präparativer HPLC: 3-(4-Fluor-phenyl-3-{6-[3-(imidazol-2-yl-amino)-propyloxy]-indol-3-yl}-propionsäure Trifluoracetat;
mit Pyridin-4-carbaldehyd und anschließender Synthesesequenz
3-Pyridin-4-yl-3-{6-[3-(imidazol-2-ylamino)-propoxy]-1H-indol-3-yl}propionsäure. Nach präparativer HPLC: 3-Pyridin-4-yl-3-{6-[3-(imidazol-2-ylamino)-propoxy]-1 H-indol-3-yl}-propionsäure Trifluoracetat;

### Beispiel 5:

### 1. 3-Phenyl-3-[6-(4-hydroxy-butyloxy)-indol-3-yl]-propionsäure-ethylester 12

Analog zu Beispiel 1.4 werden 1.2 g (3.88 mmol) 3-Phenyl-3-(6-hydroxyindol-3-yl)-propionsäure-ethylester mit 1.16 g (7.6 mmol) 4-Brom-1-butanol in Gegenwart von 2.1 g (15.2 mmol) Kaliumcarbonat in 30 ml Aceton umgesetzt. Man erhält 3-Phenyl-3-[6-(4-hydroxy-butyloxy)-indol-3-yl]-propionsäure-ethylester.

HPLC: (RP-18, Gradient A/B 99:1→1:99 in 1 h wie oben) Rₜ =43.4 min;
DC: Si-60, Toluol/Aceton 4:1, R_{f} = 0.13;
FAB-MS: (M+1) = 382.

### 2. 3-Phenyl-3-(6-{4-[(pyridin-2-yl)-(2,2,2-trichlorethoxycarbonyl)-amino]-butyloxy}-indol-3-yl)-propionsäure-ethylester 13

Die Umsetzung von 170 mg (0.45 mmol) **12** mit 178 mg (0.66 mmol) 2-(2,2,2-Trichlorethoxycarbonyl-amino)-pyridin in Gegenwart von 293 mg (0.88 mmol) Triphenylphosphin (polymergebunden) und 0.103 ml (0.66 mmol) DEAD in 6 ml THF entsprechend Beispiel 1.5 liefert nach Aufarbeitung und Chromatographie 3-Phenyl-3-(6-{4-[(pyridin-2-yl)-(2,2,2-trichlorethoxycarbonyl)-amino]-butyloxy}-indol-3-yl)-propionsäureethylester.

HPLC: (RP-18, Gradient A/B 99:1→1:99 in 1 h wie oben) Rₜ = 57.4 min;
DC: Si-60, Toluol/Aceton 4:1, R_{f} = 0.47;
FAB-MS: (M+1) = 633.

### 3. 3-Phenyl-3-{6-[4-(pyridin-2-yl-amino)-butyloxy]-indol-3-yl}-propionsäureethylester 14

Analog zu Beispiel 1.6 erhält man nach Troc-Spaltung mit Zink in Essigsäure/THF 3-Phenyl-3-{6-[4-(pyridin-2-yl-amino)-butyloxy]-indol-3-yl}propionsäure-ethylester.

HPLC: (RP-18, Gradient A/B 99:1→1:99 in 1 h wie oben) Rₜ = 44.3 min;
FAB-MS: (M+1) = 458.

### 4. 3-Phenyl-3-{6-[4-(pyridin-2-yl-amino)-butyloxy]-indol-3-yl}propionsäure 15

Analog zu Beispiel 1.7 erhält man nach basischer Ethylesterspaltung mit 1 N Natronlauge in Dioxan 3-Phenyl-3-{6-[4-(pyridin-2-yl-amino)-butyloxy]-indol-3-yl}-propionsäure. Nach präparativer HPLC 3-Phenyl-3-{6-[4-(pyridin-2-yl-amino)-butyloxy]-indol-3-yl}-propionsäure Trifluoracetat.

HPLC: (RP-18, Gradient A/B 99:1→1:99 in 1 h wie oben) Rₜ = 36.1 min;
FAB-MS: (M+1) = 430.

### Beispiel 6:

1. Analog zu Beispiel 1 erhält man durch Umsetzung von 5-Benzyloxy-indol mit Benzaldehyd und Meldrumsäure und nachfolgender Synthesesequenz 3-Phenyl-3-{5-[3-(pyridin-2-yl-amino)-propyloxy]-indol-3-yl}-propionsäure. Nach präparativer HPLC: 3-Phenyl-3-{5-[3-(pyridin-2-yl-amino}-propyloxy]-indol-3-yl}-propionsäure Trifluoracetat, Smp. 240° (Zers.).
   HPLC: (RP-18, Gradient A/B 99:1→1:99 in 1 h wie oben) Rₜ = 33.5 min;
   FAB-MS: (M+1) = 416.
2. Analog zu Beispiel 1 erhält man durch Umsetzung von 5-Benryloxy-indol mit Benzaldehyd und Meldrumsäure und nachfolgender Synthesesequenz mit 4-Brom-1-butanol 3-Phenyl-3-{5-[4-(pyridin-2-yl-amino)-butyloxy]-indol-3-yl}-propionsäure. Nach präparativer HPLC: 3-Phenyl-3-{5-[4-(pyridin-2-ylamirlo)-butyloxy]-indol-3-yl)-propionsäure Trifluoracetat.
   HPLC: (RP-18, Gradient A/B 99:1→1:99 in 1 h wie oben) Rₜ = 35.1 min;
   FAB-MS: (M+1) = 430.

### Beispiel 7:

### 1. 3-Phenyl-3-[6-(tert.butoxy-carbonylmethoxy)-indol-3-yl]-propionsäureethylester 18

Die analog zu Beispiel 1.1-1.3 hergestellte Verbindung 3-Phenyl-3-(6-hydroxy-indol-3-yl)-propionsäure-ethylester **4** (3.23 mmol) wird mit 0.94 ml (6.4 mmol) Bromessigsäure-tert.butylester und 1.8 g (13 mmol) Kaliumcarbonat in 20 ml Aceton bei 60°C über Nacht gerührt. Nach vollständiger Reaktion (DC-Kontrolle Toluol/Aceton 4:1) wird vom Rückstand abfiltriert, die Lösung eingeengt und das Rohprodukt durch Chromatographie an Kieselgel (Eluent Toluol/Aceton 9:1) gereinigt.
Man erhält 3-Phenyl-3-[6-(tert.butoxy-carbonylmethoxy)-indol-3-yl]-propionsäure-ethylester.

DC: Si-60, Toluol/Aceton 4:1, R_{f} = 0.56;
FAB-MS: (M+1) = 424.

### 2. 3-Phenyl-3-(6-carboxymethoxy-indol-3-yl)-propionsäure-ethylester 19

1 g (2,36 mmol) **18** werden in 20 mi Dichlormethan gelöst und mit 2 ml Trifluoressigsäure bei Raumtemperatur 20 h gerührt. Anschließend engt man die Lösung ein und reinigt den Rückstand durch präparative HPLC an RP-18. Man erhält 3-Phenyl-3-(6-carboxymethoxy-indol-3-yl)-propionsäureethylester Trifluoracetat.

HPLC: (RP-18, Gradient A/B 99:1→1:99 in 1 h) Rₜ = 40.72 min;
FAB-MS: (M+1) = 368.

### 3. 3-Phenyl-3-[6-(pyridin-2-yl-amidocarboxymethoxy)-indol-3-yl]-propionsäure-ethylester 20

100 mg (0.27 mmol) **19** werden mit 51 mg (0.54 mmol) 2-Amino-pyridin in Gegenwart von 112 mg (0.35 mmol) TBTU (O-(Benzotriazol-1-yl)-N,N,N',N'-tetramethyluronium-tetrafluoroborat), 11 mg (81 µmol) HOBT, (1-Hydroxy-benzotriazol Hydrat) und 90 µl (0.82 mmol) 4-Methylmorpholin in 5 ml DMF über Nacht bei Raumtemperatur gerührt. Nach vollständiger Umsetzung gießt man die Reaktionslösung auf 100 ml Wasser und extrahiert mit Ethylacetat. Nach üblicher Aufarbeitung erhält man 3-Phenyl-3-[6-(pyridin-2-yl-amidocarboxymethoxy)-indol-3-yl]-propionsäureethylester.

HPLC: (RP-18, Gradient A/B 99:1→1:99 in 1 h) Rₜ = 40.96 min;
FAB-MS: (M+1 ) = 444.

### 4. 3-Phenyl-3-[6-(pyridin-2-yl-amidocarboxymethoxy)-indol-3-yl]-propionsäure 21

Die Umsetzung von 50 mg (113 µmol) **20** mit 0.15 ml 1-N NaOH in 1 ml Dioxan liefert bei. Raumtemperatur nach 24 h 3-Phenyl-3-[6-(pyridin-2-ylamidocarboxymethoxy)-indol-3-yl]-propionsäure. Nach präparativer HPLC: 3-Phenyl-3-[6-(pyridin-2-yl-amidocarboxymethoxy)-indol-3-yl]-propionsäure Trifluoracetat.

HPLC: (RP-18, Gradient A/B 99:1→1:99 in 1 h) Rₜ = 32.1 min;
FAB-MS: (M+1) = 416.

### Beispiel 8:

1. Analog zu Beispiel 7.3 wird 3-Phenyl-3-(6-carboxymethoxy-indol-3-yl)-propionsäure-ethylester mit 2-Amino-benzimidazol umgesetzt. Man erhält nach Esterverseifung unter den Bedingungen von Beispiel 7.4 3-Phenyl-3-[6-(benzimidazol-2-yl-amidocarboxymethoxy)-indol-3-yl]-propionsäure. Nach präparativer HPLC: 3-Phenyl-3-[6-(benzimidazol-2-ylamidocarboxymethoxy}-indol-3-yl]-propionsäure Trifluoracetat.
   HPLC: (RP-18, Gradient A/B 99:1→1:99 in 1 h) Rₜ = 35.4 min;
   FAB-MS: (M+1) = 455.
2. Analog zu Beispiel 7.3 wird 3-Phenyl-3-(6-carboxymethoxy-indol-3-yl)-propionsäure-ethylester mit 2-Amino-imidazol umgesetzt. Man erhält nach Esterverseifung unter den Bedingungen von Beispiel 7.4 3-Phenyl-3-[6-(imidazol-2-yl-amidocarboxymethoxy)-indol-3-yl]-propionsäure. Nach präparativer HPLC: 3-Phenyl-3-[6-(imidazol-2-yl-amidocarboxymethoxy)-indol-3-yl]-propionsäure Trifluoracetat.
   HPLC: (RP-18, Gradient A/B 99:1→1:99 in 1 h) Rₜ = 29.3 min;
   FAB-MS: (M+1) = 405.

### Beispiel 9:

### 1. 6-(3-Benzyloxycarbonylamino-propyloxy)-indol 22

10 g (75 mmol) 6-Hydroxyindol und 21.5 g (79 mmol) 3-Benzyloxycarbonylamino-propylbromid werden in 150 ml Acetonitril gelöst und mit 31.1 g (225 mmol) Kaliumcarbonat bei 80°C 12 Std. gerührt. Nach vollständiger Umsetzung (DC-Kontrolle: Kieselgel Si-60 mit Toluol/Aceton 10:1) filtriert man vom unlöslichen Rückstand ab, engt die Lösung ein und reinigt das Produkt durch Chromatographie an Kieselgel mit Toluol/Aceton 10:1 als Eluent.

HPLC-MS: (Chromolith RP-18, Gradient A:B von 80:20→0:100 in 3.5 min mit A = Wasser + 0.01% TFA, B = Acetonitril), Rₜ = 2.13 min;
DC: Si-60, Toluol/Aceton 6:1, R_{f} = 0.31;
FAB-MS: (M+1) = 325.

### 2. 6-(3-Aminopropyloxy) indol 23

15 g (46 mmol) **22** werden in 100 ml Ethanol gelöst und bei Raumtemperatur (RT) mit 2 g Palladium/Aktivkohle (10%) unter Normaldruck hydriert. Nach 4 Std. filtriert man vom Katalysator ab und engt die Lösung ein. Das Rohprodukt kann ohne weitere Reinigung für die nächsten Umsetzungen verwendet werden.

HPLC: (RP-18, Gradient A:B von 99:1→1:99 in 1 h) Rₜ = 19.1 min;
DC: Si-60, Ethylacetat/Methanol/Wasser 4:3:2, R_{f} = 0.07;
FAB-MS: (M+1) = 191.

### 3. 6-(3-(N-Benzylpyridinium-2-yl-amino)-propyloxy)-indol Hydrobromid 24

3.5 g (18.4 mmol) **23** werden mit 5.2 g (18.4 mmol) N-Benzyl-2-chlorpyridinium Hydrobromid in Gegenwart von 11 g (129 mmol) Natriumhydrogencarbonat in 200 ml Ethanol bei RT unter Schutzgas (Stickstoff) 12 Std. gerührt. Nach beendeter Reaktion filtriert man die anorganischen Salze ab und engt die Lösung im Vakuum ein.

HPLC: (RP-18, Gradient A:B von 99:1→1:99 in 1 h) Rₜ = 35.6 min;
DC: Si-60, Dichlormethan/Methanol 6:1, R_{f} = 0.55;
FAB-MS: M⁺ = 438.

### 4. 3-[(1-(4-Fluor-phenyl)-2-(4,6-dioxo-2,2-dimethyl-[1,3]-dioxan-5-yl)-ethyl]-6-[3-(N-benzylpyridinium-2-yl-amino)-propyloxy]-indol Hydrobromid 25

500 mg (1.05 mmol) **24** werden mit 110µl (1.05 mmol) 4-Fluorbenzaldehyd, 150 mg (1.05 mmol) Meldrumsäure (2,2-Dimethyl-[1,3]-dioxan-4,6-dion) und 6 mg (0.05 mmol) L-Prolin in 4 ml Acetonitril bei 30°C 12 Std. gerührt. Nach dem Einengen der Lösung wird das Rohprodukt mit MTB-Ether (Methyl-tert-butyl-Ether) verrieben und der kristalline Rückstand abgesaugt. Dieser kann direkt weiter zur Esterspaltung und Decarboxylierung verwendet werden.

HPLC-MS: (Chromolith RP-18, Gradient A:B von 80:20→0:100 in 3.5 min mit A = Wasser + 0.01% TFA, B = Acetonitril), Rₜ = 1.77 min;
M⁺ = 608.

### 5. 3-(4-Fluorphenyl)-3-{6-[3-(N-benyzl-pyridinium-2-yl-amino)-propyloxy]-indol-3-yl}-propionsäure Trifluoracetat 26

295 mg (0.43 mmol) **25** werden in 3.5 ml DMSO gelöst und mit 36 mg (0.85 mmol) Lithiumchlorid und 9 µl Wasser 12 Std. bei 100°C gerührt. Nach beendeter Reaktion (HPLC-MS Kontrolle) wird die Lösung eingeengt und der Rückstand durch präparative HPLC an RP-18 gereinigt. Das Produkt fällt nach Gefriertrocknung der HPLC-Lösung als weißer, amorpher Feststoff in Form des Trifluoracetats an.

HPLC: (RP-18, Gradient A:B von 99:1→1:99 in 1 h) Rₜ = 38.1 min;
FAB-MS: (M⁺) = 524.

### 6. 3-(4-Fluorphenyl)-3-{6-[3-(pyridin-2-yl-amino)-propyloxy]-indol-3-yl}propionsäure 27

60 mg (94 µmol) **26** werden in 5 ml Aceton gelöst und in Gegenwart von 40 mg (0.48 mmol) Natriumhydrogencarbonat und 20 mg Palladium/Aktivkohle (10%) 10 Std. bei RT und Normaldruck hydriert. Nach Abfiltrieren des Katalysators und Einengen der Lösung erhält man 3-(4-Fluorphenyl)-3-{6-[3-(pyridin-2-yl-amino)-propyloxy]-indol-3-yl}-propionsäure. Nach präparativer HPLC an RP-18 erhält man 3-(4-Fluorphenyl)-3-{6-[3-(pyridin-2-yl-amino)-propyloxy]-indol-3-yl}-propionsäure Trifluoracetat.

HPLC: (RP-18, Gradient A:B von 99:1→1:99 in 1 h) Rₜ = 31.6 min;
FAB-MS: (M+1) = 434.

### Beispiel 10:

### Analog zu Beispiel 9 erhält man aus der Umsetzung von 6-(3-(N-Benzylpyridinium-2-yl-amino)-propyloxy)-indol Hydrobromid 24

mit 3,5-Dichlor-benzaldehyd und anschließender Synthesesequenz
3-(3,5-Dichlorphenyl)-3-{6-[3-(pyridin-2-yl-amino)-propyloxy]-indol-3-yl}propionsäure. Nach präparativer HPLC: 3-(3,5-Dichlorphenyl)-3-{6-[3-(pyridin-2-yl-amino)-propyloxy]-indol-3-yl}-propionsäure Trifluoracetat;

HPLC: (RP-18, Gradient A:B von 99:1→1:99 in 1 h) Rₜ = 37.2 min;
FAB-MS: (M+1) = 485;
mit 4,6-Dichlor-benzaldehyd und anschließender Synthesesequenz
3-(4,6-Dichlorphenyl)-3-{6-[3-(pyridin-2-yl-amino)-propyloxy]-indol-3-yl}propionsäure. Nach präparativer HPLC: 3-(4,6-Dichlorphenyl)-3-{6-[3-(pyridin-2-yl-amino)-propyloxy]-indol-3-yl}-propionsäure Trifluoracetat;

HPLC: (RP-18, Gradient A:B von 99:1→1:99 in 1 h) Rₜ = 37.3 min;
FAB-MS: (M+1) = 485;
mit 4-Chlor-5-trifluormethyl-benzaldehyd und anschließender Synthesesequenz
3-(4-Chlor-5-trifluormethyl-phenyl)-3-{6-[3-(pyridin-2-yl-amino)-propyloxy]-indol-3-yl}-propionsäure. Nach präparativer HPLC: 3-(4-Chlor-5-trifluormethyl-phenyl)-3-{6-[3-(pyridin-2-yl-amino)-propyloxy]-indol-3-yl}propionsäure Trifluoracetat;

HPLC: (RP-18, Gradient A:B von 99:1→1:99 in 1 h) Rₜ = 38.7 min;
FAB-MS: (M+1) = 518;
mit 3-Cyclohexyl-benzaldehyd und anschließender Synthesesequenz
3-Cyclohexyl-3-{6-[3-(pyridin-2-yl-amino)-propyloxy]-indol-3-yl}propionsäure. Nach präparativer HPLC: 3-Cyclohexyl-3-{6-[3-(pyridin-2-ylamino)-propyloxy]-indol-3-yl}-propionsäure Trifluoracetat;

HPLC: (RP-18, Gradient A:B von 99:1→1:99 in 1 h) Rₜ = 37.2 min;
FAB-MS: (M+1) = 422;
mit Benzo[1,2,5]thiadiazol-5-carbaldehyd und anschließender Synthesesequenz
3-Benzo[1,2,5]thiadiazol-5-yl-3-{6-[3-(pyridin-2-ylamino)-propoxy]-1Hindol-3-yl}-propionsäure. Nach präparativer HPLC: 3-3-Benzo[1,2,5]thiadiazol-5-yl-3-{6-[3-(pyridin-2-ylamino)-propoxy]-1H-indol-3-yl}-propionsäure Trifluoracetat;

HPLC: (RP-18, Gradient A:B von 99:1→1:99 in 1 h) Rₜ = 30.7 min;
FAB-MS: (M+1) = 474;
mit 2,6-Difluor-benzaldehyd und anschließender Synthesesequenz
3-(2,6-Difluorphenyl)-3-{6-[3-(pyridin-2-yl-amino)-propyloxy]-indol-3-yl}propionsäure. Nach präparativer HPLC: 3-(2,6-Difluorphenyl)-3-{6-[3-(pyridin-2-yl-amino)-propyloxy]-indol-3-yl}-propionsäure Trifluoracetat;

HPLC: (RP-18, Gradient A:B von 99:1→1:99 in 1 h) Rₜ = 32.6 min;
FAB-MS: (M+1) = 452;
mit 2-Chlor-3,6-difluor-benzaldehyd und anschließender Synthesesequenz
3-(2-Chlor-3,6-Difluorphenyl)-3-{6-[3-(pyridin-2-yl-amino)-propyloxy]-indol-3-yl}-propionsäure. Nach präparativer HPLC: 3-(2-Chlor-3,6-Difluorphenyl)-3-{6-[3-(pyridin-2-yl-amino)-propyloxy]-indol-3-yl}propionsäure Trifluoracetat;

HPLC: (RP-18, Gradient A:B von 99:1→1:99 in 1 h) Rₜ = 34.6 min;
FAB-MS: (M+1) = 486;
mit 2,4,6-Trifluor-benzaldehyd und anschließender Synthesesequenz
3-(2,4,6-Trifluorphenyl)-3-{6-[3-(pyridin-2-yl-amino)-propyloxy]-indol-3-yl}propionsäure. Nach präparativer HPLC: 3-(2,4,6-Trifluorphenyl)-3-{6-[3-(pyridin-2-yl-amino)-propyloxy]-indol-3-yl}-propionsäure Trifluoracetat;

HPLC: (RP-18, Gradient A:B von 99:1→1:99 in 1 h) Rₜ = 33.8 min;
FAB-MS: (M+1) = 470.

### Beispiel 11:

### 1. 6-[3-(Pyridin-2-yl-amino)-propyloxy]-indol 28

6 g (12.9 mmol) 6-(3-(N-Benzylpyridinium-2-yl-amino)-propyloxy)-indol Hydrobromid **24** [hergestellt analog zu Beispiel 9.1-9.3] werden in 300 ml Aceton gelöst und in Gegenwart von 2 g Palladium/Aktivkohle (10%) 8 h bei RT und Normaldruck hydriert. Nach Filtration vom Katalysator wird die Lösung eingeengt und das Rohprodukt als weißer Feststoff erhalten.

DC: Si-60, Dichlormethan/Methanol 6:1, R_{f} = 0.67;
HPLC: (RP-18, Gradient A:B von 99:1→1:99 in 1 h) Rₜ = 28.6 min;
FAB-MS: (M+1) = 268.

### 2. 3-[(1-(4-Trifluormethoxy-phenyl)-2-(4,6-dioxo-2,2-dimethyl-[1,3]-dioxan-5-yl)-ethyl]-6-[3-(pyridin-2-yl-amino)-propyloxy]-indol 29

350 mg (1.3 mmol) **28** werden mit 190 µl (1.3 mmol) 4-Trifluormethoxy-benzaldehyd, 190 mg (1.3 mmol) Meldrumsäure und 9 mg (0.07 mmol) Prolin in 5 ml Acetonitril bei RT 12 Std. gerührt. Nach beendeter Reaktion (Kontrolle durch HPLC-MS) engt man die Lösung ein und setzt das Produkt ohne weitere Reinigung zur Esterspaltung und Decarboxylierung ein.

HPLC-MS: (Chromolith RP-18, Gradient A:B von 80:20→0:100 in 3.5 min mit A = Wasser + 0.01% TFA, B = Acetonitril), Rₜ = 1.71 min;
(M+1 ) = 544.

### 3. 3-(4-Trifluormethoxy-phenyl)-3-{6-[3-(pyridin-2-yl-amino)-propyloxy]-indol-3-yl}-propionsäure Trifluoracetat 30

Entsprechend Beispiel 9.5 werden 760 mg (1.3 mmol) **29** in 4 ml DMSO mit 110 mg Lithiumchlorid und 29 µl Wasser bei 100°C 12 Std. gerührt. Nach vollständiger Umsetzung engt man die Lösung ein und erhält 3-(4-Trifluormethoxy-phenyl)-3-{6-[3-(pyridin-2-yl-amino)-propyloxy]-indol-3-yl}propionsäure. Nach präp. HPLC an RP-18 erhält man 3-(4-Trifluormethoxyphenyl)-3-{6-[3-(pyridin-2-yl-amino)-propyloxy]-indol-3-yl}-propionsäure Trifluoracetat.

HPLC: (RP-18, Gradient A:B von 99:1→1:99 in 1 h) Rₜ = 36.7 min;
FAB-MS: (M+1) = 498.

### Analog zu Beispiel 11 erhält man aus der Umsetzung von 6-[3-(Pyridin-2-ylamino)-propyloxy]-indol 28

mit 3-Trifluormethoxy-benzaldehyd und anschließender Synthesesequenz
3-(3-Trifluormethoxy-phenyl)-3-{6-[3-(pyridin-2-yl-amino)-propyloxy]-indol-3-yl}-propionsäure. Nach präparativer HPLC erhält man 3-(3-Trifluormethoxy-phenyl)-3-{6-[3-(pyridin-2-yl-amino)-propyloxy]-indol-3-yl}propionsäure Trifluoracetat;

HPLC: (RP-18, Gradient A:B von 99:1→1:99 in 1 h) Rₜ = 40.2 min;
FAB-MS: (M+1) = 500.

### Beispiel 12:

### 1. 6-[3-(4,5-Dihydro-1H-imidazol-2-yl-amino)-propyloxy]-indol 31

500 mg (2.6 mmol) 6-(3-Aminopropyloxy) indol **23** [hergestellt nach Beispiel 9.1] werden mit 0.97 g ( 3.9 mmol) 2-(3,5-Dimethylpyrazolyl)-4,5-dihydroimidazol Hydrobromid und 1.7 ml (11.9 mmol) Triethylamin in 10 ml DMF gelöst und bei 60°C 12 Std. gerührt. Nach dem Einengen der Lösung wird das Rohprodukt durch präp. HPLC gereinigt.

HPLC: (RP-18, Gradient A:B von 99:1→1:99 in 1 h) Rₜ = 26.7 min;
FAB-MS: (M+1) = 259.

### 2. 3-[(1-(Benzo[1,2,5]thiadiazol-5-yl)-2-(4,6-dioxo-2,2-dimethyl-[1,3]-dioxan-5-yl)-ethyl]-6-[3-(4,5-dihydro-1H-imidazol-2-yl-amino)-propyloxy]-indol 32

Entsprechend Beispiel 11.2 werden 100 mg (0.33 mmol) **31** mit 53 mg (0.33 mmol) 5-Formyl-benzo[1,2,5]thiadiazol, 46 mg (0.33 mmol) Meldrumsäure und 2 mg L-Prolin in 4 ml Acetonitril bei 30°C umgesetzt. Nach Einengen erhält man einen Rückstand, der ohne Reinigung weiter umgesetz wird.

HPLC-MS: (Chromolith RP-18, Gradient A:B von 80:20→0:100 in 3.5 min mit A = Wasser + 0.01% TFA. B = Acetonitril), Rₜ = 1.29 min; M+1 = 549.

### 3. 3-(Benzo[1,2,5]thiadiazol-5-yl)-3-{6-[3-(4,5-dihydro-1H-imidazol-2-ylamino)-propyloxy]-indol-3-yl}-propionsäure 33

Das Rohprodukt **32** wird in 4 ml DMSO mit 27 mg Lithiumchlorid und 7 µl Wasser 12 Std. bei 100°C gerührt, dann eingeengt. Man erhält 3-(Benzo[1,2,5]thiadiazol-5-yl)-3-{6-[3-(4,5-dihydro-1*H*-imidazol-2-yl-amino)-propyloxy]-indol-3-yl}-propionsäure. Nach Reinigung durch präp. HPLC erhält man 3-(Benzo[1,2,5]thiadiazol-5-yl)-3-{6-[3-(4,5-dihydro-1*H*-imidazol-2-yl-amino)-propyloxy]-indol-3-yl}-propionsäure Trifluoracetat;

HPLC: (RP-18, Gradient A:B von 99:1→1:99 in 1 h) Rₜ = 28.1 min;
FAB-MS: (M+1) = 465.

### Beispiel 13:

### Analog zu Beispiel 12 erhält man aus der Umsetzung von 6-[3-(4,5-Dihydro-1H-imidazol-2-yl-amino)-propyloxy]-indol 31

mit 4-Fluor-benzaldehyd und anschließender Synthesesequenz
3-{6-[3-(4,5-Dihydro-1H-imidazol-2-ylamino)-propoxy]-1H-indol-3-yl}-3-(4-fluor-phenyl)-propionsäure. Nach präparativer HPLC erhält man 3-{6-[3-(4,5-Dihydro-1H-imidazol-2-ylamino)-propoxy]-1H-indol-3-yl}-3-(4-fluorphenyl)-propionsäure Trifluoracetat;
mit Benzaldehyd und anschließender Synthesesequenz
3-{6-[3-(4,5-Dihydro-1H-imidazol-2-ylamino)-propoxy]-1H-indol-3-yl}-3-phenyl-propionsäure. Nach präparativer HPLC erhält man 3-{6-[3-(4,5-Dihydro-1H-imidazol-2-ylamino)-propoxy]-1H-indol-3-yl}-3-phenylpropionsäure Trifluoracetat;

HPLC: (RP-18, Gradient A:B von 99:1→1:99 in 1 h) Rₜ = 29.8 min;
FAB-MS: (M+1) = 407;
mit Pyridin-4-carbaldehyd und anschließender Synthesesequenz
3-Pyridin-4-yl-3-{6-[3-(4,5-dihydro-1H-imidazol-2-ylamino)-propoxy]-1Hindol-3-yl}-propionsäure. Nach präparativer HPLC: 3-Pyridin-4-yl-3-{6-[3-(3,5-dihydro-1H-imidazol-2-ylamino)-propoxy]-1H-indol-3-yl}-propionsäure Trifluoracetat.

Analog zu Beispiel 12 erhält man aus der Umsetzung von 6-[3-(4,5-Dihydro-1H-imidazol-2-yl-amino)-butyloxy]-indol, hergestellt analog zu Beispiel 9.1-9.3 durch Umsetzung mit 4-Benzyloxycarbonylaminobutylbromid

### Beispiel 14:

### 3-Phenyl-3-{6-[3-(1,5-dihydro-imidazol-4-on-2-yl-amino)-propyloxy]-indol-3-yl}-propionsäure 38

130 mg (0.29 mmot) **36,** hergestellt analog zu Beispiel 14, werden mit 115 mg (0.87 mmol) 2-Methylsulfariyl-1,5-dihydro-imidazot-4-on und 0.12 ml (0.87 mmot) Triethylamin in einem Gemisch aus 2ml Ethanol und 1 ml DMF bei RT 24 Std. gerührt. Man erhält 3-Phenyl-3-{6-[3-(1,5-dihydro-imidazot-4-on-2-yl-amino)-propyloxy]-indol-3-yl}-propionsäure. Nach Reinigung durch präp. HPLC an RP-18 erhält man 3-Phenyl-3-{6-[3-(1,5-dihydroimidazol-4-on-2-yl-amino)-propyloxy]-indol-3-yl}-propionsäure Trifluoracetat.
FAB-MS: (M+1) = 421.

### Beispiel 15:

Analog zu Beispiel 16 erhält man aus der Umsetzung von 3-(4-Fluorphenyl)-3-[6-(3-amino-propyloxy)-indol-3-yl]-propionsäure (hergestellt analog zu Beispiel 1.1-1.2 und 15) mit 2-Methylsulfanyl-1,5-dihydroimidazol-4-on und anschließender Synthesesequenz
3-(4-Fluor-phenyl)-3-{6-[3-(4-oxo-4,5-dihydro-1H-imidazol-2-ylamino)-propoxy]-1H-indol-3-yl}-propionsäure. Nach präparativer HPLC erhält man 3-(4-Fluor-phenyl)-3-{6-[3-(4-oxo-4,5-dihydro-1H-imidazol-2-ylamino)-propoxy]-1 H-indol-3-yl}-propionsäure Trifluoracetat.

Analog zu Beispiel 16 erhält man aus der Umsetzung von 3-[6-(3-Aminopropoxy)-1 H-indol-3-yl]-3-pyridin-4-yl-propionsäure (hergestellt analog zu Beispiel 1.1-1.2 und 15) mit 2-Methylsulfanyl-1,5-dihydro-imidazol-4-on und anschließender Synthesesequenz
3-{6-[3-(4-Oxo-4,5-dihydro-1H-imidazol-2-ylamino)-propoxy]-1H-indol-3-yl}-3-pyridin-4-yl-propionsäure. Nach präparativer HPLC erhält man 3-{6-[3-(4-Oxo-4,5-dihydro-1H-imidazol-2-ylamino)-propoxy]-1H-indol-3-yl}-3-pyridin-4-yl-propionsäure Trifluoracetat.

Analog zu Beispiel 16 erhält man aus der Umsetzung von 3-[6-(3-Aminopropoxy)-1*H*-indol-3-yl]-3-benzo[1,2,5]thiadiazol-5-yl-propionsäure (hergestellt analog zu Beispiel 1.1-1.2 und 15) mit 2-Methylsulfanyl-1,5-dihydro-imidazol-4-on und anschließender Synthesesequenz
3-Benzo[1,2,5]thiadiazol-5-yl-3-{6-[3-(4-oxo-4,5-dihydro-1H-imidazol-2-ylamino)-propoxy]-1H-indol-3-yl}-propionsäure. Nach präparativer HPLC erhält man 3-Benzo[1,2,5]thiadiazol-5-yl-3-{6-[3-(4-oxo-4,5-dihydro-1Himidazol-2-ylamino)-propoxy]-1H-indol-3-yl}-propionsäure Trifluoracetat.

### Beispiel 16:

### 3-Phenyl-3-{6-[3-(pyrimidin-2-yl-amino)-propyloxy]-indol-3-yl}-propionsäureethylester 39

1 g (2.48 mmol) **35**, hergestellt nach Beispiel 14.2, werden mit 426 mg (3.72 mmol) 2-Chlorpyrimidin und 1 ml (7.44 mmol) Triethylamin in 30 ml wasserfreiem Ethanol gelöst und 20 Std. unter Rückfluß gekocht. Nach dem Einengen wird der Rückstand an Kieselgel chromatographiert (Eluent Ethylacetat).

DC: Si-60, Ethylacetat, R_{f} = 0.42;
HPLC: (RP-18, Gradient A:B von 99:1→1:99 in 1 h) Rₜ = 39.0 min;
FAB-MS: (M+1) = 445.

Durch Esterspaltung mit Natronlauge in Dioxan bei RT wird die freie Säure 3-Phenyl-3-{6-[3-(pyrimidin-2-yl-amino)-propyloxy]-indol-3-yl}-propionsäure erhalten.
FAB-MS: (M+1) = 417.

### Beispiel 17:

Analog zu Beispiel 9 wird Verbindung **24** mit 3-Hydroxybenzaldehyd und nachfolgender Synthesesequenz zu 3-(3-Hydroxy-phenyl)-3-{6-[3-(pyridin-2-ylamino)-propoxy]-1H-indol-3-yl}-propionsäuremethylester umgesetzt.

### Beispiel 18:

### 1. (3S)-3-Phenyl-3-{6-[3-(pyridin-2-yl-amino)-propyloxy]-indol-3-yl}propionsäure 46

50 g (0.113 mol) 3-Phenyl-3-{6-[3-(pyridin-2-yl-amino)-propyloxy]-indol-3-yl}-propionsäure-ethylester **7**, hergestellt nach Beispiel 1, werden durch kontinuierliche Chromatographie an einem modifizierten Celluloseträger (Chiralcel OD-H) in Isopropanol/n-Heptan 30:70 in die beiden Enantiomeren getrennt.
Ausbeute: 24.5 g (98% d. Th.) des aktiven S-Enantiomers.
HPLC: Chiralcel OD-H, iPropanol/n-Heptan 30/70, Rₜ = 14.08 min.

Zur Esterspaltung werden 24.4 g (55 mmol) des S-Eantiomers in 100 ml Ethanol gelöst und bei 60°C mit 110 ml (110 mmol) 1 N NaOH 12 Std. gerührt. Nach vollständiger Umsetzung läßt man die Reaktionslösung abkühlen und säuert mit 1 N HCl auf pH 6 an. Der ausfallende Niederschlag wird abgesaugt, mit Wasser und anschließend mit MTB-Ether gewaschen und getrocknet. Man erhält (3S)-3-Phenyl-3-{6-[3-(pyridin-2-yl-amino)-propyloxy]-indol-3-yl}-propionsäure.

Smp.: 137°C;
HPLC: (RP-18, Gradient A:B von 99:1→1:99 in 1 h mit A=Wasser+0.3%TFA, B=Acetonitril/ Wasser+0.3% TFA 4:1) Rₜ = 31.1 min; chirale HPLC: Chirobiotic V, Wasser (+ 1%Triethylammoniumacetat)/Methanol 65:35, Rₜ = 21.15 min.

### 2. (3S)-3-Phenyl-3-{6-[3-(pyridin-2-yl-amino)-propyloxy]-indol-3-yl}propionsäure Hydrochlorid

2 g (4.8 mmol) des inneren Salzes **46** werden in 5 ml Dioxan gelöst und mit 20 ml (20 mmol) 1 N HCl 2 Std. bei RT gerührt. Anschließend wird die Lösung gefriergetrocknet. Man erhält (3S)-3-Phenyl-3-{6-[3-(pyridin-2-ylamino)-propyloxy]-indol-3-yl}-propionsäure Hydrochlorid.

| | | | | | |
|---|---|---|---|---|---|
| Analyse | berechnet | 66.4% C, | 5.80% H, | 9.30% N, | 7.84% Cl |
| | gefunden | 65.9% C, | 5.91% H, | 9.11% N, | 7.44% Cl. |

### 3. (3S)-3-Phenyl-3-{6-[3-(pyridin-2-yl-amino)-propyloxy]-indol-3-yl}propionsäure Methansulfonat

2 g (4.8 mmol) des inneren Salzes **46** werden in 5 ml Dioxan gelöst und mit 310 µl (4.8 mmol) Methansulfonsäure in 5 ml Wasser bei RT2 Std. gerührt. Anschließend engt man die Lösung ein und gewinnt (3S)-3-Phenyl-3-{6-[3-(pyridin-2-yl-amino)-propyloxy]-indol-3-yl}-propionsäure Methansulfonat nach Gefriertrocknung aus Acetonitril/Wasser.

| | | | | | |
|---|---|---|---|---|---|
| Analyse | berechnet | 61.04% C, | 5.71% H, | 8.21% N, | 6.26% S |
| | gefunden | 60.90% C, | 5.99% H, | 8.01 % N, | 5.92% S. |

Die nachfolgenden Beispiele betreffen pharmazeutische Zubereitungen:

### Beispiel A: Injektionsgläser

Eine Lösung von 100 g eines Wirkstoffes der Formel 1 und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel 1, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 I isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Indot-3-yl-Derivate der Formel I 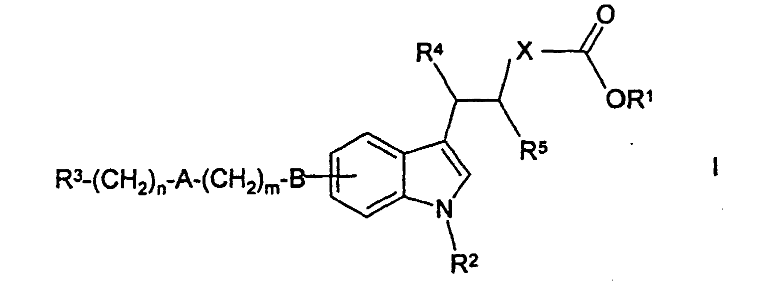 worin
A NH, CONH, NHCO oder eine direkte Bindung sein kann,
B O ist,
X eine direkte Bindung,
R¹ H,
R² H,
R³ 1 H-Imidazol-2-yl, 4,5-Dihydro-imidazol-2-yl, 3,5-Dihydro-imidazol-4-on-2-yl oder Pyridin-2-yl, wobei diese jeweils ein- oder zweifach durch =O oder NHZ substituiert sein können,
R⁴ Phenyl, 3-Trifluormethoxyphenyl, 4-Fluorphenyl, 3-Chlorphenyl, 3-Hydroxyphenyl, Pyridin-4-yl, 3,5-Dichlorphenyl, 2,4-Dichlorphenyl, Cyclohexyl, 4-Chlor-3-trifluormethylphenyl, Benzothiadiazol-5-yl, 2,6-Difluorphenyl, 2-Chlor-3,6-difluorphenyl, 2,4,6-Trifluorphenyl oder Cyclohexyl
R⁵ H,
Z Alkyl mit 1 bis 6 C-Atomen,
n 0,
m 3 oder 4
bedeuten,
sowie deren physiologisch unbedenklichen Salze und Solvate.

2. Enantiomere der Formel 1 gemäß Anspruch 1.

3. Verbindungen der Formel I gemäß Anspruch 1
a) 3-Phenyl-3-{6-[3-(pyridin-2-ylamino)-propoxy]-1H-indol-3-yl}propionsäure;
b) 3-Phenyl-3-{6-[3-(pyridin-2-ylamino)-propoxy]-1H-indol-3-yl}propionsäure;
c) 3-Phenyl-3-{6-[4-(pyridin-2-ylamino)-butoxy]-1H-indol-3-yl}propionsäure;
d) 3-Phenyl-3-{5-[4-(pyridin-2-ylamino)-butoxy]-1H-indol-3-yl}propionsäure;
e) 3-Phenyl-3-{5-[3-(pyridin-2-ylamino)-propoxy]-1H-indol-3-yl}propionsäure;
f) 3-Phenyl-3-[6-(pyridin-2-yl-amidocarboxymethoxy)-indol-3-yl]-propionsäure;
g) 3-Phenyl-3-[6-(benzimidazol-2-yl-amidocarboxymethoxy)-indol-3-yl]-propionsäure;
h) 3-Phenyl-3-[6-(imidazol-2-yl-amidocarboxymethoxy)-indol-3-yl]-propionsäure;
i) 3-{6-[3-(4,5-Dihydro-1H-imidazol-2-ylamino)-propoxy]-1H-indol-3-yl}-3-phenyl-propionsäure:
j) 3-(4-Fluorphenyl)-3-{6-[3-(pyridin-2-yl-amino)-propyloxy]-indol-3-yl}propionsäure;
k) 3-(3,5-Dichlorphenyl)-3-{6-[3-(pyridin-2-yl-amino)-propyloxy]-indol-3-yl}-propionsäure;
l) 3-(4-Chlor-5-trifluormethyl-phenyl)-3-{6-[3-(pyridin-2-yl-amino)-propyloxy]-indol-3-yl}-propionsäure;
m) 3-Cyclohexyl-3-{6-[3-(pyridin-2-yl-amino)-propyloxy]-indol-3-yl}propionsäure;
n) 3-Pyridin-4-yl-3-{6-[3-(pyridin-2-ylamino)-propoxy]-1H-indol-3-yl}propionsäure;
o) 3-(3-Chlor-phenyl-3-{6-[3-(pyridin-2-yl-amino)-propyloxy]-indol-3-yl}propionsäure;
p) 3-Phenyl-3-[6-(3-guanidino-propyloxy)-indol-3-yl]-propionsäure;
q) 3-Benzo[1,2,5]thiadiazol-5-yl-3-{6-[3-(pyridin-2-ylamino)-propoxy]-1Hindol-3-yl}-propionsäure;
r) 3-(3-Hydroxy-phenyl)-3-{6-[3-(3,4,5,6-tetrahydro-pyridin-2-ylamino)-propoxy]-1H-indol-3-yl}-propionsäure oder
s) 3-[4-Methoxycarbonyl-phenyl]-3-{6-[3-(pyridin-2-yl-amino)-propyloxy]-indol-3-yl}-propionsäure,
sowie deren physiologisch unbedenklichen Salze und Solvate.

4. Verfahren zur Herstellung von Verbindungen der Formel I nach Anspruch 1 sowie ihrer Salze oder Solvate, **dadurch gekennzeichnet, daß** man
a) eine Verbindung der Formel I aus einem ihrer funktionellen Derivate durch Behandeln mit einem solvolysierenden oder hydrogenolysierenden Mittel in Freiheit setzt,
oder
b) daß man einen Rest R¹, R², R³, R⁴, R⁵ und/oder R⁶ in einen anderen Rest R¹, R², R³, R⁴, R⁵ und/oder R⁶ umwandelt, indem man beispielsweise
i) eine Aminogruppe durch Umsetzung mit einem amidierenden Mittel in eine Guanidinogruppe umwandelt,
ii) einen Ester verseift,
iii) eine Aminogruppe alkyliert oder acyliert,
iv) eine Cyangruppe in eine Amidinogruppe umwandelt,
und/oder eine Base oder Säure der Formel I in eines ihrer Salze umwandelt.

5. Verbindungen der Formel I nach Anspruch 1 und ihre physiologisch unbedenklichen Salze oder Solvate als therapeutische Wirkstoffe.

6. Pharmazeutische Zubereitung **gekennzeichnet durch** einen Gehalt an mindestens einer Verbindung der Formel I nach Anspruch 1 und/oder einem ihrer physiologisch unbedenklichen Salze oder Solvate.

7. Verwendung von Verbindungen der Formel I nach Anspruch 1 und/oder ihre physiologisch unbedenklichen Salze oder Solvate zur Herstellung eines Arzneimittels.

8. Verwendung von Verbindungen der Formel 1, nach Anspruch 7 und/oder ihrer physiologisch unbedenklichen Salze oder Solvate zur Herstellung eines Arzneimittels zur Behandlung von Thrombosen, Herzinfarkt, koronaren Herzerkrankungen, Arteriosklerose, Entzündungen, Restenose, rheumatischer Arthritis, makularer degenerativer Krankheit, diabetischer Retinopathie, Tumorerkrankungen, Osteoporose, Infektionen und Restenose nach Angioplastie.

9. Verbindungen der Formel IIa 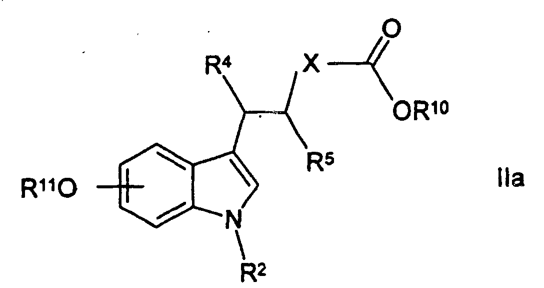 worin R², R⁴ oder R⁵ eine in Anspruch 1 angegebene Bedeutung haben und
X eine Bindung und
R¹⁰ und R¹¹ jeweils unabhängig voneinander eine Hydroxyschutzgruppe oder H bedeuten.

10. Verbindungen der Formel X 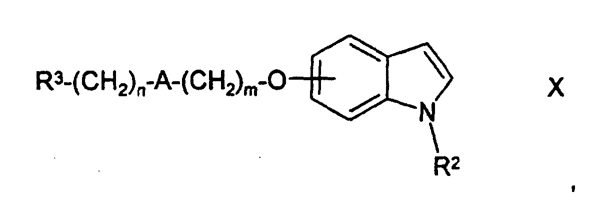 worin
R², R³, A, n und m eine in Anspruch 1 angegebene Bedeutung haben sowie deren Salze.

## Claims

1. Indol-3-yl derivatives of the formula I 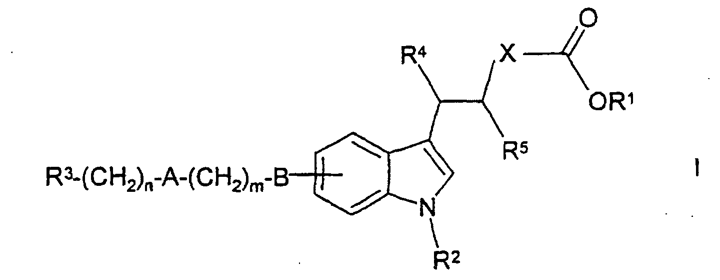 in which
A can be NH, CONH, NHCO or a direct bond,
B is O,
X denotes a direct bond,
R¹ denotes H,
R² denotes H,
R³ denotes 1 H-imidazol-2-yl, 4,5-dihydroimidazol-2-yl, 3,5-dihydroimidazol-4-on-2-yl or pyridin-2-yl, where these may each be mono- or disubstituted by =O or NHZ,
R⁴ denotes phenyl, 3-trifluoromethoxyphenyl, 4-fluorophenyl, 3-chlorophenyl, 3-hydroxyphenyl, pyridin-4-yl, 3,5-dichlorophenyl, 2,4-dichlorophenyl, cyclohexyl, 4-chloro-3-trifluoromethylphenyl, benzothiadiazol-5-yl, 2,6-difluorophenyl, 2-chloro-3,6-difluorophenyl, 2,4,6-trifluorophenyl or cyclohexyl
R⁵ denotes H,
Z denotes alkyl having 1 to 6 C atoms,
n denotes 0,
m denotes 3 or 4,
and physiologically acceptable salts and solvates thereof.

2. Enantiomers of the formula I according to Claim 1.

3. Compounds of the formula I according to Claim 1
a) 3-phenyl-3-{6-[3-(pyridin-2-ylamino)propoxy]-1H-indol-3-yl}-propionic acid;
b) 3-phenyl-3-{6-[3-(pyridin-2-ylamino)propoxy]-1H-indol-3-yl}-propionic acid;
c) 3-phenyl-3-{6-[4-(pyridin-2-ylamino)butoxy]-1H-indol-3-yl}propionic acid;
d) 3-phenyl-3-{5-[4-(pyridin-2-ylamino)butoxy]-1H-indol-3-yl}propionic acid;
e) 3-phenyl-3-{5-[3-(pyridin-2-ylamino)propoxy]-1H-indol-3-yl}-propionic acid;
f) 3-phenyl-3-[6-(pyridin-2-ylamidocarboxymethoxy)indol-3-yl]-propionic acid;
g) 3-phenyl-3-[6-(benzimidazol-2-ylamidocarboxymethoxy)indol-3-yl]-propionic acid;
h) 3-phenyl-3-[6-(imidazol-2-ylamidocarboxymethoxy)indol-3-yl]-propionic acid;
i) 3-{6-[3-(4,5-dihydro-1H-imidazol-2-ylamino)propoxy]-1H-indol-3-yl}-3-phenylpropionic acid:
j) 3-(4-fluorophenyl)-3-{6-[3-(pyridin-2-ylamino)propoxy]indol-3-yl}-propionic acid;
k) 3-(3,5-dichlorophenyl)-3-{6-[3-(pyridin-2-ylamino)propoxy]indol-3-yl}propionic acid;
l) 3-(4-chloro-5-trifluoromethylphenyl)-3-{6-[3-(pyridin-2-ylamino)-propoxy]indol-3-yl}propionic acid;
m) 3-cyclohexyl-3-{6-[3-(pyridin-2-ylamino)propoxy]indol-3-yl}-propionic acid;
n) 3-pyridin-4-yl-3-{6-[3-(pyridin-2-ylamino)propoxy]-1H-indol-3-yl}-propionic acid;
o) 3-(3-chlorophenyl-3-{6-[3-(pyridin-2-ylamino)propoxy]indol-3-yl}-propionic acid;
p) 3-phenyl-3-[6-(3-guanidinopropoxy)indol-3-yl]propionic acid;
q) 3-benzo-1,2,5-thiadiazol-5-yl-3-{6-[3-(pyridin-2-ylamino)propoxy]-1 H-indol-3-yl}propionic acid;
r) 3-(3-hydroxyphenyl)-3-{6-[3-(3,4,5,6-tetrahydropyridin-2-ylamino)-propoxy]-1H-indol-3-yl}propionic acid or
s) 3-[4-methoxycarbonylphenyl]-3-{6-[3-(pyridin-2-ylamino)propoxy]-indol-3-yl}propionic acid;
and physiologically acceptable salts and solvates thereof.

4. Process for the preparation of compounds of the formula I according to Claim 1 and salts or solvates thereof, **characterised in that**
a) a compound of the formula I is liberated from one of its functional derivatives by treatment with a solvolysing or hydrogenolysing agent,
or
b) **in that** a radical R¹, R², R³, R⁴, R⁵ and/or R⁶ is converted into another radical R¹, R², R³, R⁴, R⁵ and/or R⁶ by, for example,
i) converting an amino group into a guanidino group by reaction with an amidating agent,
ii) saponifying an ester,
iii) alkylating or acylating an amino group,
iv) converting a cyano group into an amidino group,
and/or a base or acid of the formula I is converted into one of its salts.

5. Compounds of the formula I according to Claim 1 and physiologically acceptable salts or solvates thereof as therapeutic active ingredients.

6. Pharmaceutical composition **characterised by** a content of at least one compound of the formula I according to Claim 1 and/or one of its physiologically acceptable salts or solvates.

7. Use of compounds of the formula I according to Claim 1 and/or physiologically acceptable salts or solvates thereof for the preparation of a medicament.

8. Use of compounds of the formula I according to Claim 7 and/or physiologically acceptable salts or solvates thereof for the preparation of a medicament for the treatment of thromboses, cardiac infarction, coronary heart diseases, arteriosclerosis, inflammation, restenosis, rheumatic arthritis, macular degenerative disease, diabetic retinopathy, tumour diseases, osteoporosis, infections and restenosis after angioplasty.

9. Compounds of the formula IIa 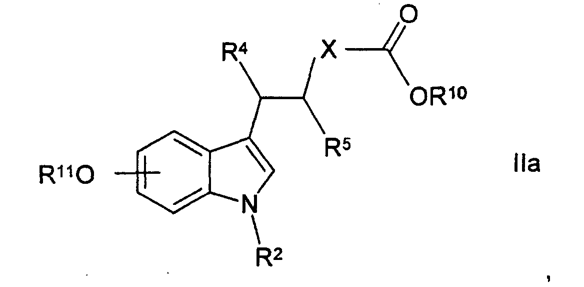 in which R², R⁴ or R⁵ have a meaning indicated in Claim 1 and X denotes a bond and
R¹⁰ and R¹¹ each, independently of one another, denote a hydroxyl-protecting group or H.

10. Compounds of the formula X 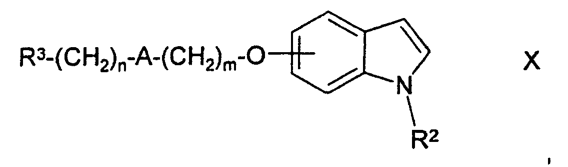 in which
R², R³, A, n and m have a meaning indicated in Claim 1, and salts thereof.

## Revendications

1. Dérivés d'indol-3-yle de la formule 1 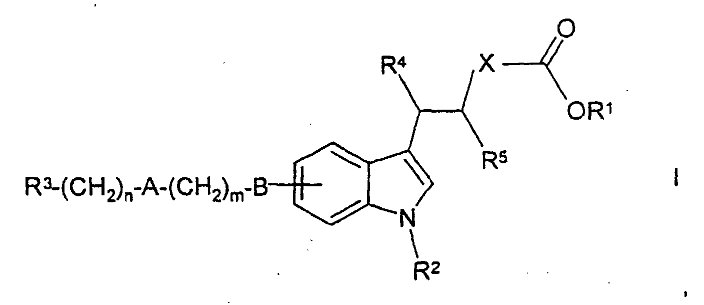 dans laquelle
A peut être NH, CONH, NHCO ou une liaison directe,
B est O,
X représente une liaison directe,
R¹ représente H,
R² représente H,
R³ représente 1H-imidazol-2-yle, 4,5-dihydroimidazol-2-yle, 3,5-dihydroimidazol-4-on-2-yle ou pyridine-2-yle, où chacun peut être mono- ou disubstitué par =O ou NHZ,
R⁴ représente phényle, 3-trifluorométhoxyphényle, 4-fluorophényle, 3-chlorophényle, 3-hydroxyphényle, pyridine-4-yle, 3,5-dichlorophényle, 2,4-dichlorophényle, cyclohexyle, 4-chloro-3-trifluorométhylphényle, benzothiadiazol-5-yle, 2,6-difluorophényle, 2-chloro-3,6-difluorophényle, 2,4,6-trifluorophényle ou cyclohexyle,
R⁵ représente H,
Z représente alkyle comportant de 1 à 6 atomes de C,
n représente 0,
m représente 3 ou 4,
et leurs sels et solvates physiologiquement acceptables.

2. Enantiomères de la formule 1 selon la revendication 1.

3. Composés de la formule 1 selon la revendication 1
a) acide 3-phényl-3-{6-[3-(pyridine-2-ylamino)propoxy]-1H-indo(-3-yl}-propionique;
b) acide 3-phényl-3-{6-[3-(pyridine-2-ylamino)propoxy]-1H-indol-3-yl}-propionique;
c) acide 3-phényl-3-{6-[4-(pyridine-2-ylamino)butoxy]-1H-indol-3-yl}-propionique;
d) acide 3-phényl-3-{5-[4-(pyridine-2-ylamino)butoxy]-1H-indol-3-yl}-propionique;
e) acide 3-phényl-3-{5-[3-(pyridine-2-ylamino)propoxy]-1H-indol-3-yl}-propionique;
f) acide 3-phényl-3-[6-(pyridine-2-ylamidocarboxyméthoxy)indol-3-yl}propionique;
g) acide 3-phény(-3-[6-(benzimidazol-2-ylamidocarboxyméthoxy)-indol-3-yl}-propionique;
h) acide 3-phényl-3-[6-(imidazol-2-ylamidocarboxyméthoxy)indol-3-yl}propionique;
i) acide 3-{6-[3-(4,5-dihydro-1H-imidazol-2-ylamino)propoxy]-1H-indol-3-yl}-3-phénylpropionnque
j) acide 3-(4-fluorophényl)-3-{6-[3-(pyridine-2-ylamino)propoxy]indol-3-yl}propionique;
k) acide 3-(3,5-dichlorophényl)-3-{6-[3-(pyridine-2-ylamino)propoxy]-indol-3-yl}-propionique;
l) acide 3-(4-chloro-5-trifluorométhylphényl)-3-{6-[3-(pyridine-2-yl-amino)propoxy]indol-3-yl}-propionique;
m) acide 3-cyclohexyl-3-{6-[3-(pyridine-2-ylamino)propoxyjindol-3-yl}-propionique;
n) acide 3-pyridine-4-yl-3-{6-[3-(pyridine-2-ylamino)propoxy]-1H-indol-3-yl}propionique;
o) acide 3-(3-chlorophényl-3-{6-[3-(pyridine-2-ylamino)propoxy]indol-3-yl}propionique;
p) acide 3-phényl-3-[6-(3-guanidinopropoxy)indol-3-yl}propionique;
q) acide 3-benzo-1,2,5-thiadiazol-5-yl-3-{6-[3-(pyridine-2-ylamino)-propoxy]-1H-indol-3-yl}propionique;
r) acide 3-(3-hydroxyphényl)-3-{6-[3-(3,4,5,6-tétrahydropyridine-2-yl-amino)propoxy]-1H-indol-3-yl}propionique ou
s) acide 3-[4-méthoxycarbonylphényl]-3-{6-[3-(pyridine-2-ylamino)-propoxy]indol-3-yl}propionique;
et leurs sels et solvates physiologiquement acceptables.

4. Procédé pour la préparation de composés de la formule I selon la revendication 1 et de leurs sels ou solvates, **caractérisé en ce que**
a) un composé de la formule I est libéré à partir d'un de ses dérivés fonctionnels par traitement avec un agent de solvolyse ou d'hydrogénolyse,
ou
b) **en ce qu'**un radical R¹, R², R³, R⁴, R⁵ et/ou R⁶ est converti selon un autre radical R¹, R², R³, R⁴, R⁵ et/ou R⁶ en, par exemple,
i) convertissant un groupe amino selon un groupe guanidino par réaction avec un agent d'amidification,
ii) saponifiant un ester,
iii) alkylatant ou acylatant un groupe amino,
iv) convertissant un groupe cyano selon un groupe amidino,
et/ou une base ou un acide de la formule I est converti selon l'un de ses sels.

5. Composés de la formule 1 selon la revendication 1 et leurs sels ou solvates physiologiquement acceptables en tant qu'ingrédients thérapeutiques actifs.

6. Composition pharmaceutique **caractérisée par** une teneur en au moins un composé de la formule I selon la revendication 1 et/ou en au moins un de ses sels ou solvates physiologiquement acceptables.

7. Utilisation de composés de la formule 1 selon la revendication 1 et/ou de leurs sels ou solvates physiologiquement acceptables pour la préparation d'un médicament.

8. Utilisation de composés de la formule 1 selon la revendication 7 et/ou de leurs sels ou solvates physiologiquement acceptables pour la préparation d'un médicament pour le traitement des thromboses, des infarctus cardiaques, des maladies coronariennes du coeur, de l'artériosclérose, des inflammations, de la resténose, de l'arthrite rhumatique, de la maladie dégénérative maculaire, de la rétinopathie diabétique, des maladies tumorales, de l'ostéoporose, des infections et de la resténose après angioplastie.

9. Composés de la formule IIa 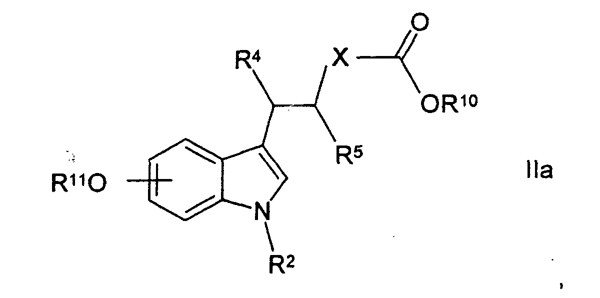 dans laquelle R², R⁴ ou R⁵ présentent une signification indiquée dans la revendication 1 et
X représente une liaison et
R¹⁰ et R¹¹, chacun indépendamment l'un de l'autre, représentent un groupe de protection des hydroxyles ou H.

10. Composés de la formule X 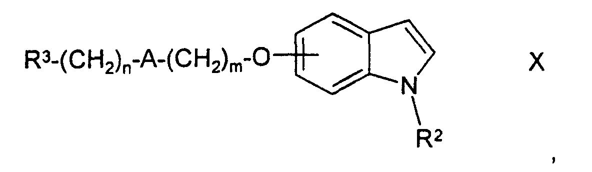 dans laquelle
R², R³, A, n et m présentent une signification indiquée dans la revendication 1, et leurs sels.
